# EUROPEAN PATENT SPECIFICATION

(11) **EP 1 819 812 B1**
(45) Date of publication and mention of the grant of the patent: **30.05.2012**
(21) Application number: 05815880.9
(22) Date of filing: 01.12.2005
(51) Int. Cl.: C12N 9/02, C12Q 1/66, C12N 15/62, G01N 33/533

(54) **THE LUPAC BIFUNCTIONAL MARKER AND ITS USE IN PROTEIN PRODUCTION**
DER LUPAC BIFUNCTIONNELLE MARKER UND DESSEN GEBRAUCH IN DER HERSTELLUNG VON PROTEINEN
MARQUEUR BIFONCTIONNEL LUPAC ET SON UTILISATION POUR LA PRODUCTION DE PROTEINES

(30) Priority: 02.12.2004 EP 04106251; 06.12.2004 US 633637 P
(43) Date of publication of application: 22.08.2007
(73) Proprietor: Merck Serono SA, 1267 Coinsins, Vaud (CH)
(72) Inventor: CHATELLARD, Philippe, CH-1005 Lausanne (CH); IMHOF, Markus, CH-1071 Chexbres (CH)
(74) Representative: Merck Serono SA - Geneva Intellectual Property
(86) International application number: PCT/EP2005/056373
(87) International publication number: WO 2006/058900

(56) References cited:
- EP-A- 1 262 553
- WO-A-2004/081167

## Description

### FIELD OF THE INVENTION

This invention relates to industrial production of proteins. More specifically, the invention relates to surrogate markers corresponding to a fusion between luciferase and the puromycin N-acetyl transferase. The invention further relates to the use of these surrogate for screening cells for high expression of a protein of interest.

### BACKGROUND

Introducing heterologous genes into animal host cells and screening for expression of the added genes is a lengthy and complicated process. Typically a number of hurdles have to be overcome: (i) the construction of large expression vectors; (ii) the transfection and selection of clones with stable long-term expression, eventually in the absence of selective pressure; and (iii) screening for high expression rates of the heterologous protein of interest.

### 1. Selection of clones expressing the heterologous gene

Selection of the clones having integrated the gene of interest is performed using a selection marker conferring resistance to a selective pressure. Most of the selection markers confer resistance to an antibiotic such as, e.g., neomycin, kanamycin, hygromycin, gentamycin, chloramphenicol, puromycin, zeocin or bleomycin.

When generating cell clones expressing a gene of interest from expression vectors, host cells are typically transfected with a plasmid DNA vector encoding both the protein of interest and the selection marker on the same vector. Quite often the capacity of a plasmid is limited and the selection marker has to be expressed from a second plasmid, which is co-transfected with the plasmid comprising the gene of interest.

Stable transfection leads to random integration of the expression vector in the genome of the host cell. Use of selective pressure, e.g. by administrating an antibiotic to the media, will eliminate all cells that did not integrate the vector containing the selection marker providing resistance to the respective antibiotic or selective pressure. If this selection marker is on the same vector as the gene of interest or, if this selection marker is on a second vector and vector comprising the gene of interest was co-integrated, the cells will express both the selection marker and the gene of interest. It is frequently observed, however, that the expression level of the gene of interest is highly variable depending on the site of integration. Furthermore, when removing selective pressure, expression becomes quite often very unstable or even extinguished. Only a small number of initial transfectants are thus providing high and stable long-term expression and it is extremely tedious to identify these clones in a large population of candidates. Typically, high expressing candidates are isolated and then cultivated in absence of selective pressure. Under these conditions a large proportion of initially selected candidates are eliminated due to their loss of gene of interest expression upon removal of selective pressure. It would thus be advantageous to cultivate the candidates, following an initial period of selection for stable transfection, in absence of selective pressure and only then screen for gene of interest expression.

### 2. Screening for high expressing clones

Screening for high-expressing clones for a protein of interest is often done by methods directly revealing the presence of high amounts of the protein. Typically immunologic methods, such as ELISA or immunohistochemical staining, are applied to detect the product either intracellularly or in cell culture supernatants. These methods are tedious, expensive, time-consuming, and often not amenable to high throughput screenings (HTS). In addition, an antibody reactive to the expressed protein must be available.

Attempts to quantify the protein amounts by Fluorescence-Activated Cell Sorting (FACS) have also been made, but only with a limited success, especially in the case of secreted proteins (Borth et al., 2000)

One approach for the screening of high expression rates of the protein of interest would be the use of an easily measurable surrogate marker, expressed from the same vector as the gene of interest (Chesnut *et al*., 1996). The idea underling the use of a measurable surrogate marker is that there is a correlation between the expression of the gene of interest and the surrogate marker gene due to the physical link of the two genes on the same vector.

Numerous easily measurable markers are available in the art. They usually correspond to enzymes which act on a chromogenic or luminogenic substrate such as, e.g., the β-glucuronidase, the chloramphenicol acetyltransferase, the nopaline synthase, the β-galactosidase, secreted alkaline phosphatase (SEAP) and the luciferase. The green fluorescent protein (GFP) may also be used as a measurable marker in FACS. The activity of all these proteins can be measured by standard assays that may be used in HTS.

The drawback of this approach is the use of yet another expression cassette for the surrogate marker gene. This renders the expression vector rather bulky, hosting expression units comprising a promoter, a cDNA and polyadanylation signals for at least three proteins (i.e., the gene of interest, the selection marker and the surrogate marker). For multi-chain proteins the situation becomes even more complex. Alternatively, individual plasmid vectors expressing the three genes, which encode the protein of interest, the selection marker and the surrogate marker respectively, could be co-transfected. However, it is likely that the vectors would be either integrated at different loci, or exhibit varying and uncorrelated expression.

A promising approach for overcoming the above limitations consists in the use of a chimeric marker that combines the functional properties of a selection marker and of a measurable marker. Such an approach has been described by, e.g., Bennett *et al.* (1998). This article discloses the GFP-Zeo^{R} marker, which confers resistance to the Zeocin antibiotic, and the expression of which can be monitored by fluorescence microscopy.

EP 1 262 553 discloses chimeric markers and their use either in a method for trapping unknown genes, or in a method of selecting cells in which a genetic element has been targeted into a predefined locus by homologous recombination. However, EP 1 262 553 does not teach the use of chimeric markers for screening for clones expressing high levels of a recombinant protein. Furthermore, the experimental data relates to a chimeric marker corresponding to a fusion protein between luciferase and the protein conferring resistance to hygromycin.

The potential use of chimeric markers for screening for high-expressing clones remains a poorly explored field, and the efficiency of such chimeric markers for screening for high-expressing clones needs to be further investigated. The finding of a novel, alternative and powerful chimeric surrogate marker would be extremely useful in the field of industrial production of therapeutic proteins.

### SUMMARY OF THE INVENTION

The present invention stems from the construction and characterization of a novel bifunctional chimeric marker, Lupac. Lupac corresponds to a fusion protein between luciferase and a protein conferring resistance to puromycin, the puromycin N-acetyl transferase (pac). It has been demonstrated that Lupac combines the functional properties of both luciferase and pac. Lupac's usefulness for the isolation of high-expressing clones for a therapeutic protein has further been demonstrated. Therefore, a first aspect of the invention relates to a Lupac polypeptide comprising a fragment of a luciferase fused to a fragment of a puromycin N-acetyl transferase (pac), wherein:
a) said Lupac polypeptide exhibits:
   (i) luciferase activity; and
   (ii) puromycin N-acetyl transferase activity.
b) said fragment of a luciferase comprises an amino acid sequence at least 95% identical to a *Photinus pyralis* luciferase of SEQ ID NO: 8, and
c) said fragment of a pac comprises an amino acid sequence at least 95% identical to a *Streptomyces alboniger* pac of SEQ ID NO: 9.

A second aspect relates to a nucleic acid encoding a Lupac polypeptide according to the invention.

A third aspect relates to a vector comprising a nucleic acid according to the invention.

A fourth aspect relates to a cell comprising a nucleic acid according to the invention.

A fifth aspect relates to the use of a cell comprising a nucleic acid according to the invention for producing a protein of interest.

A sixth aspect relates to the use of a polypeptide, a nucleic acid or a vector according to the invention for screening cells for expression of a protein of interest.

A seventh aspect relates to a method of screening cells for expression of a protein of interest, said method comprising the step of:
(i) transfecting cells by a an expression vector according to the invention;
(ii) selecting cells being resistant to puromycin; and
(iii) assaying the luciferase activity of the cells selected in step (ii).

An eight aspect relates to a method of obtaining a cell line expressing a protein of interest, said method comprising the step of:
(i) screening cells according to a method of screening according to the invention;
(ii) selecting the cell exhibiting the highest expression of said protein of interest; and
(iii) establishing a cell line from said cell.

A ninth aspect relates to a method of producing a protein of interest, said method comprising the step of:
(i) culturing a cell line obtained according to the invention under conditions which permit expression of said protein of interest; and
(ii) collecting said protein of interest.

A tenth aspect relates to a method of producing a polypeptide according to the invention comprising the step of:
(i) culturing a cell comprising a nucleic acid according to the invention under conditions which permit expression of the Lupac polypeptide; and
(ii) collecting the polypeptide according to the invention.

### BRIEF DESCRIPTION OF THE FIGURES

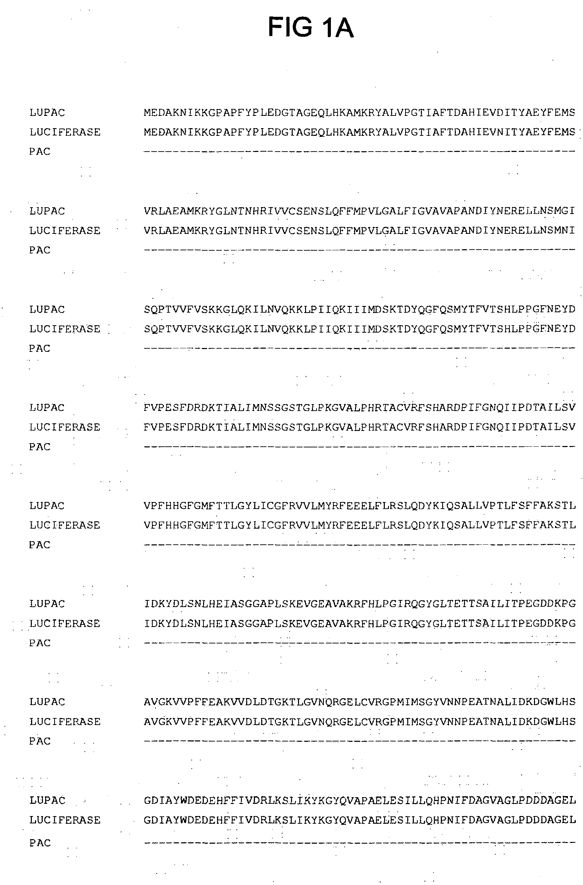

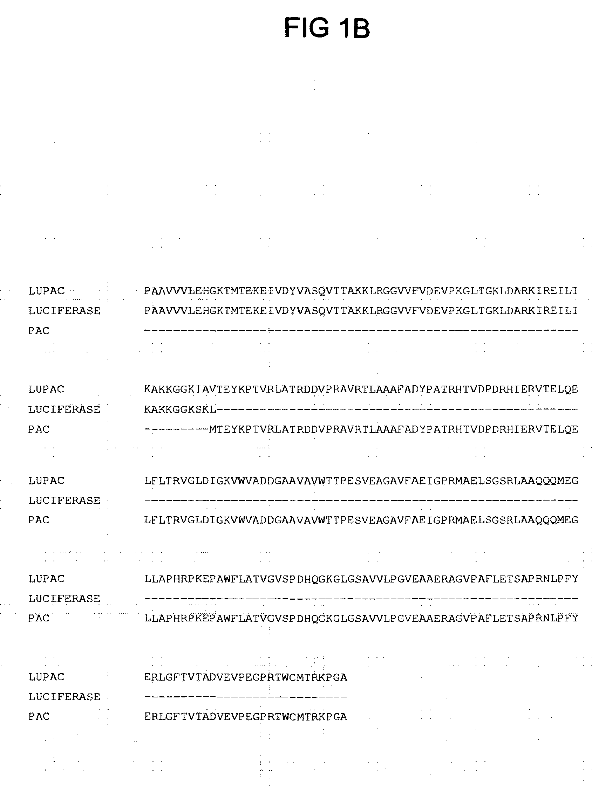

Figure 1 shows an alignment between a Lupac polypeptide in accordance with the invention (SEQ ID NO: 2), luciferase (SEQ ID NO: 8) and pac (SEQ ID NO: 9).
Figure 2 shows a scheme of the pGlupac and of the pBSI.IL18BPmCMVLupac.I vectors. pGlupac contains the ORF for a Lupac polypeptide of SEQ ID NO: 1 expressed form an SV40 promoter with an SV40 enhancer at 3'. Plasmid pBSI.IL18BPmCMVLupac.I contains a Lupac polypeptide of SEQ ID NO: 2 expressed from the IE1 promoter of the bi-directional mouse CMV immediate early region. The IE2 promoter of this vector is driving expression of IL18BP.
Figure 3 shows the luciferase activity of CHO cells transiently transfected either with pGlupac or with pGL3ctrl + pPur (vectors comprising luciferase and pac respectively). Luciferase activity is normalized by cell density in million/ml.
Figure 4 shows the positive correlation between expression of a Lupac polypeptide of SEQ ID NO: 2 (RLU) and IL 18BP (RU) in 24 clones transfected with the pBSI.IL18BPmCMVLupac.I vector.
Figure 5 is a scheme of the expression vector used in Example 4. This vector contains a Lupac polypeptide of SEQ ID NO: 2 expressed from the IE1 promoter of the bi-directional mouse CMV immediate early region. The IE2 promoter of this vector is driving expression of a recombinant protein referred to as "Serono Protein 1" (r-SP1). The insulator is described in PCT/EP2004/052591.
Figures 6 and 7 show the r-SP1 titer and the viable cell density obtained when culturing a CHO cell line expressing r-SP1 (CHO-r-SP1), which was selected using Lupac as a surrogate marker (see Example 4).

### BRIEF DESCRIPTION OF THE SEQUENCES OF THE SEQUENCE LISTING

SEQ ID NO: 1 corresponds to the nucleic acid sequence of a Lupac polypeptide in accordance with the invention.
SEQ ID NO: 2 corresponds to the protein sequence encoding a Lupac polypeptide in accordance with the invention.
SEQ ID Nos. 3 to 6 correspond to primers used for constructing a Lupac polypeptide in accordance with the invention.
SEQ ID NO: 7 corresponds to the fragment of the mouse CMV immediate early region present in the pBSI.IL18BPmCMVLupac.I vector.
SEQ ID NO: 8 corresponds to the protein sequence of the *photinus pyralis* luciferase.
SEQ ID NO: 9 corresponds to the protein sequence of the *Streptomyces alboniger* pac.

### DETAILED DESCRIPTION OF THE INVENTION

The present invention stems from the construction and characterization of a novel bifunctional chimeric marker referred to as Lupac. Lupac corresponds to a fusion protein between luciferase and a protein conferring resistance to puromycin, the puromycin N-acetyl transferase (pac).

It has been demonstrated that Lupac combines the functional properties of luciferase and of pac (Example 2). Accordingly, the Lupac marker can be used both as a selectable marker in stable transfections due to its pac activity and as an easily measurable surrogate marker due to its luciferase activity.

Lupac's usefulness for the isolation of high-expressing clones for a therapeutic protein has further been demonstrated. In Example 3, a vector comprising Lupac and a gene of interest, expressed from two different promoters, has been constructed. It has been shown that there is a very good positive correlation between Lupac expression levels and expression levels of the gene of interest.

Accordingly, the present invention provides a powerful marker, Lupac, which can both be used to provide selectivity in stable transfection and act as a surrogate marker for screening candidate clones for high expression of a gene of interest. Using Lupac in HTS allows keeping the same chance for selecting high-expressing clones as when the expression level of the gene of interest is measured directly. Moreover, using Lupac allows reducing time, cost and resources since (i) standardized product-independent and simple analysis is performed; and (ii) measuring luciferase activity is an inexpensive assay.

### 1. Lupac polypeptides

A first aspect of the present invention relates to a polypeptide comprising a fragment of a luciferase fused to a fragment of a puromycin N-acetyl transferase (pac), wherein
a) said Lupac polypeptide exhibits:
   (i) luciferase activity; and
   (ii) puromycin N-acetyl transferase activity.
b) said fragment of a luciferase comprises an amino acid sequence at least 95% identical to a *Photinus pyralis* luciferase of SEQ ID NO: 8, and
c) said fragment of a pac comprises an amino acid sequence at least 95% identical to a *Streptomyces alboniger* pac of SEQ ID NO: 9.

As further used herein, the term "a Lupac polypeptide" or "Lupac" refers to such a polypeptide.

As used herein, a polypeptide exhibits "luciferase activity" when said polypeptide is capable of oxidizing luciferin. Preferably, said polypeptide is capable of catalyzing at least one of the following reactions:
- Photinus luciferin + O₂ + ATP => oxidized Photinus luciferin + CO₂ + H₂O + AMP + diphosphate + light.
- Renilla or Cypridina luciferin + O₂ <=> oxidized Renilla luciferin + CO₂ + light Photinus luciferin refers to (S)-4,5-dihydro-2-(6-hydroxy-2- benzothiazoloyl)-4-thiazolecarboxylic acid. Cypridina luciferin refers to [3-[3,7-dihydro-6-(1H-indol-3-yl)-2-[(S)-1- methyl-6-propyl]-3-oxoimidazo-[1,2-a]pyrazin-8-yl]propyl]guanidine. Renilla luciferin refers to 8-benzyl-2-(4-hydroxybenzyl)-6-(4-hydroxyphenyl)imidazo-[1,2-A]pyrazin-3(7H)-one.

Measurement of the light emitted during the above reactions allows measurement of luciferase activity. The luciferase activity can for example be measured as described in Example 2.2.

As used herein, a polypeptide exhibits "puromycin N-acetyl transferase activity" when said polypeptide is capable of conferring resistance to puromycin to a cell. The puromycin N-acetyl transferase activity can for example be measured as described in Example 2.3.

Herein described is a polypeptide that comprises a fragment of a luciferase coming from a firefly such as, e.g., *photinus pyralis, Luciola cruciata, Luciola lateralis* or *Photuris pennsylvanica.* The Lupac polypeptide comprises a fragment of the *photinus pyralis* luciferase. As used herein, the term *"photinus pyralis* luciferase" refers to a polypeptide of SEQ ID NO: 8, or to an allelic variant, a splice variant or a mutein thereof. Most preferably, said fragment of a *photinus pyralis* luciferase comprises amino acids 1 to 547 of SEQ ID NO: 8. Alternatively, said fragment of a *photinus pyralis* luciferase can correspond to a fragment of at least 50, 75, 100, 125, 150, 175, 200, 225, 250, 275, 300, 325, 350, 375, 400, 425, 450, 475, 500 or 525 amino acids of the full-length *photinus pyralis* luciferase, or to the full-length *photinus pyralis* luciferase.

Herein described is a polypeptide that comprises a fragment of a luciferase coming from *Renilla reniformis* (sea pansy) or from *Vargula hilgendorfil* (Sea firefly).

Herein described is a polypeptide that comprises a fragment of a pac coming from a Streptomyces species such as, e.g., *Streptomyces alboniger* or *Streptomyces coelicolor.* The Lupac polypeptide comprises a fragment of a *Streptomyces alboniger* pac. As used herein, the term *"Streptomyces alboniger* pac" refers to a polypeptide of SEQ ID NO: 9 or to an allelic variant, a splice variant or a mutein thereof. More Preferably, the pac fragment comprises amino acids 2 to 199 of SEQ ID NO: 9. Alternatively, said fragment of a *Streptomyces alboniger* pac can correspond to a fragment of at least 50, 75, 100, 125, 150 or 175 amino acids of the full-length *Streptomyces alboniger* pac, or to the full-length *Streptomyces alboniger* pac.

In a Lupac polypeptide, the luciferase fragment may be fused to the 5' terminus of the pac fragment, or the pac fragment may be fused to the 5' terminus of the luciferase fragment. Preferably, the luciferase fragment is fused to the 5' terminus of the pac fragment.

In a most preferred embodiment, the Lupac polypeptide comprises or consists of SEQ ID NO: 2.

In another most preferred embodiment, the Lupac polypeptide comprises or consists of an amino acid sequence at least 95%, 96%, 97%, 98% or 99% identical to SEQ ID NO: 2.

As used herein, the term "mutein" refers to an analog of a naturally occurring polypeptide, in which one or more of the amino acid residues of a naturally occurring polypeptide are replaced by different amino acid residues, or are deleted, or one or more amino acid residues are added to the naturally occurring sequence of the polypeptide, without lowering considerably the activity of the resulting products as compared with the naturally occurring polypeptide. These muteins are prepared by known synthesis and/or by site-directed mutagenesis techniques, or any other known technique suitable therefore. Muteins of *Streptomyces alboniger* pac or of *photinus pyralis* luciferase that can be used in accordance with the present invention, or nucleic acids encoding the muteins, including a finite set of substantially corresponding sequences as substitution peptides or polynucleotides which can be routinely obtained by one of ordinary skill in the art, without undue experimentation, based on the teachings and guidance presented herein.

Muteins of *Streptomyces alboniger* pac or of *photinus pyralis* luciferase in accordance with the present invention include proteins encoded by a nucleic acid, such as DNA or RNA, which hybridizes to DNA or RNA, which encodes pac or luciferase, in accordance with the present invention, under moderately or highly stringent conditions. The term "stringent conditions" refers to hybridization and subsequent washing conditions, which those of ordinary skill in the art conventionally refer to as "stringent". See Ausubel et al., Current Protocols in Molecular Biology, supra, Interscience, N.Y., §6.3 and 6.4 (1987, 1992), and Sambrook et al. (Sambrook, J. C., Fritsch, E. F., and Maniatis, T. (1989) Molecular Cloning: A Laboratory Manual, Cold Spring Harbor Laboratory Press, Cold Spring Harbor, NY).

Without limitation, examples of stringent conditions include washing conditions 12-20°C below the calculated Tm of the hybrid under study in, *e.g*., 2 x SSC and 0.5% SDS for 5 minutes, 2 x SSC and 0.1% SDS for 15 minutes; 0.1 x SSC and 0.5% SDS at 37°C for 30-60 minutes and then, a 0.1 x SSC and 0.5% SDS at 68°C for 30-60 minutes. Those of ordinary skill in this art understand that stringency conditions also depend on the length of the DNA sequences, oligonucleotide probes (such as 10-40 bases) or mixed oligonucleotide probes. If mixed probes are used, it is preferable to use tetramethyl ammonium chloride (TMAC) instead of SSC.

Muteins of *Streptomyces alboniger* pac or of *photinus pyralis* luciferase include polypeptides having an amino acid sequence at least 50% identical, more preferably at least 60% identical, and still more preferably at least 70%, 75%, 80%, 85%, 90%, 95%, 96%, 97%, 98% or 99% identical to the naturally occurring polypeptide.

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% (5 of 100) of the amino acid residues in the subject sequence may be inserted, deleted, or substituted with another amino acid.

For sequences where there is not an exact correspondence, a "% identity" may be determined. In general, the two sequences to be compared are aligned to give a maximum correlation between the sequences. This may include inserting "gaps" in either one or both sequences, to enhance the degree of alignment. A % identity may be determined over the whole length of each of the sequences being compared (so-called global alignment), that is particularly suitable for sequences of the same or very similar length, or over shorter, defined lengths (so-called local alignment), that is more suitable for sequences of unequal length.

Methods for comparing the identity and homology of two or more sequences are well known in the art. Thus for instance, programs available in the Wisconsin Sequence Analysis Package, version 9.1 (Devereux *et al.,* 1984), for example the programs BESTFIT and GAP, may be used to determine the % identity between two polynucleotides and the % identity and the % homology between two polypeptide sequences. BESTFIT uses the "local homology" algorithm of (Smith and Waterman, 1981) and finds the best single region of similarity between two sequences. Other programs for determining identity and/or similarity between sequences are also known in the art, for instance the BLAST family of programs (Altschul *et al.,* 1990), accessible through the home page of the NCBI at world wide web site ncbi.nlm.nih.gov) and FASTA (Pearson and Lipman, 1988; Pearson, 1990).

Preferred changes for muteins in accordance with the present invention are what are known as "conservative" substitutions. Conservative amino acid substitutions of *Streptomyces alboniger* pac or of *photinus pyralis* luciferase, may include synonymous amino acids within a group which have sufficiently similar physicochemical properties that substitution between members of the group will preserve the biological function of the molecule (Grantham, 1974). It is clear that insertions and deletions of amino acids may also be made in the above-defined sequences without altering their function, particularly if the insertions or deletions only involve a few amino acids, e.g. under thirty, and preferably under ten, and do not remove or displace amino acids which are critical to a functional conformation, e.g. cysteine residues. Proteins and muteins produced by such deletions and/or insertions come within the purview of the present invention.

Preferably, the synonymous amino acid groups are those defined in Table I. More preferably, the synonymous amino acid groups are those defined in Table II; and most preferably the synonymous amino acid groups are those defined in Table III.

**Table I**

| **Preferred Groups of Synonymous Amino Acids** | |
|---|---|
| *Amino Acid* | *Synonymous Group* |
| Ser | Ser, Thr, Gly, Asn |
| Arg | Arg, Gln, Lys, Glu, His |
| Leu | Ile, Phe, Tyr, Met, Val, Leu |
| Pro | Gly, Ala, Thr, Pro |
| Thr | Pro, Ser, Ala, Gly, His, Gln, Thr |
| Ala | Gly, Thr, Pro, Ala |
| Val | Met, Tyr, Phe, Ile, Leu, Val |
| Gly | Ala, Thr, Pro, Ser, Gly |
| Ile | Met, Tyr, Phe, Val, Leu, Ile |
| Phe | Trp, Met, Tyr, Ile, Val, Leu, Phe |
| Tyr | Trp, Met, Phe, Ile, Val, Leu, Tyr |
| Cys | Ser, Thr, Cys |
| His | Glu, Lys, Gln, Thr, Arg, His |
| Gin | Glu, Lys, Asn, His, Thr, Arg, Gln |
| Asn | Gln, Asp, Ser, Asn |
| Lys | Glu, Gln, His, Arg, Lys |
| Asp | Glu, Asn, Asp |
| Glu | Asp, Lys, Asn, Gln, His, Arg, Glu |
| Met | Phe, Ile, Val, Leu, Met |
| Trp | Trp |

**Table II**

| **More Preferred Groups of Synonymous Amino Acids** | |
|---|---|
| *Amino Acid* | *Synonymous Group* |
| Ser | Ser |
| Arg | His, Lys, Arg |
| Leu | Leu, Ile, Phe, Met |
| Pro | Ala, Pro |
| Thr | Thr |
| Ala | Pro, Ala |
| Val | Val, Met, Ile |
| Gly | Gly |
| Ile | Ile, Met, Phe, Val, Leu |
| Phe | Met, Tyr, Ile**,** Leu, Phe |
| Tyr | Phe, Tyr |
| Cys | Cys, Ser |
| His | His, Gln, Arg |
| Gln | Glu, Gln, His |
| Asn | Asp, Asn |
| Lys | Lys, Arg |
| Asp | Asp, Asn |
| Glu | Glu, Gln |
| Met | Met, Phe, Ile, Val, Leu |
| Trp | Trp |

**Table III**

| **Most Preferred Groups of Synonymous Amino Acids** | |
|---|---|
| *Amino Acid* | *Synonymous Group* |
| Ser | Ser |
| Arg | Arg |
| Leu | Leu, Ile, Met |
| Pro | Pro |
| Thr | Thr |
| Ala | Ala |
| Val | Val |
| Gly | Gly |
| Ile | Ile, Met, Leu |
| Phe | Phe |
| Tyr | Tyr |
| Cys | Cys, Ser |
| His | His |
| Gln | Gln |
| Asn | Asn |
| Lys | Lys |
| Asp | Asp |
| Glu | Glu |
| Met | Met, Ile, Leu |
| Trp | Trp |

Examples of production of amino acid substitutions in proteins which can be used for obtaining muteins of *Streptomyces alboniger* pac or of *photinus pyralis* luciferase for use in the present invention include any known method steps, such as presented in US patents 4,959,314, 4,588,585 and 4,737,462, to Mark et al; 5,116,943 to Koths et al., 4,965,195 to Namen et al; 4,879,111 to Chong et al; and 5,017,691 to Lee et al; and lysine substituted proteins presented in US patent No. 4,904,584 (Shaw et al).

Preferably, the muteins of the present invention exhibit substancially the same biological activity as the naturally occurring polypeptide to which it corresponds.

### 2. Lupac nucleic acids, and vectors and host cells comprising them

A second aspect of the present invention relates to a nucleic acid encoding a Lupac polypeptide. As further used in this specification, the term "Lupac nucleic acid" refers to such a nucleic acid.

In a preferred embodiment, the Lupac nucleic acid comprises or consists of SEQ ID NO: 1.

Any procedures known in the art can be used to obtain Lupac nucleic acids of the present invention. Lupac nucleic acids can for example be obtained as described in Example 1.

A third aspect of the present invention relates to a vector comprising a Lupac nucleic acid. Such a vector is referred to as a "Lupac vector" within the present specification. Preferably, the Lupac vector is an expression vector. Such a vector is further referred to as a "Lupac expression vector". The term "Lupac vector" encompasses the term "Lupac expression vector". The term "vector" is used herein to designate either a circular or a linear DNA or RNA compound, which is either doublestranded or single-stranded, and which comprise at least one polynucleotide of the present invention to be transferred in a cell host or in a unicellular or multicellular host organism. An "expression vector" comprises appropriate signals in the vectors, said signals including various regulatory elements, such as enhancers/promoters from viral, bacterial, plant, mammalian, and other eucaryotic sources that drive expression of the inserted polynucleotide in host cells.

In a most preferred embodiment, the Lupac expression vector further comprises a nucleic acid encoding a protein of interest. As shown in example 3, such vectors are particularly useful for screening cells for high expression of a protein of interest.

In accordance with the present invention, the protein of interest may be any polypeptide for which production is desired. The protein of interest may find use in the field of pharmaceutics, agribusiness or furniture for research laboratories. Preferred proteins of interests find use in the field of pharmaceutics.

For example, the protein of interest may be, e.g., a naturally secreted protein, a normally cytoplasmic protein, a normally transmembrane protein, or a human or a humanized antibody. When the protein of interest is a normally cytoplasmic or a normally transmembrane protein, the protein has preferably been engineered in order to become soluble. The polypeptide of interest may be of any origin. Preferred polypeptides of interest are of human origin.

In preferred embodiments, the protein of interest is selected from the group consisting of chorionic gonadotropin, follicle-stimulating hormone, lutropin-choriogonadotropic hormone, thyroid stimulating hormone, human growth hormone, interferons (e.g., interferon beta-1a, interferon beta-1b), interferon receptors (e.g., interferon gamma receptor), TNF receptors p55 and p75, interleukins (e.g., interieukin-2, interleukin-11), interleukin binding proteins (e.g., interleukin-18 binding protein), anti-CD11a antibodies, erythropoietin, granulocyte colony stimulating factor, granulocyte-macrophage colony-stimulating factor, pituitary peptide hormones, menopausal gonadotropin, insulin-like growth factors (e.g., somatomedin-C), keratinocyte growth factor, glial cell line-derived neurotrophic factor, thrombomodulin, basic fibroblast growth factor, insulin, Factor VIII, somatropin, bone morphogenetic protein-2, platelet-derived growth factor, hirudin, epoietin, recombinant LFA-3/IgG1 fusion protein, glucocerebrosidase, and muteins, fragments, soluble forms, functional derivatives, fusion proteins thereof.

In a preferred embodiment, the Lupac expression vector is a nucleic acid encoding a protein of interest and comprising at least two promoters, one driving the expression of the Lupac polypeptide, and the other one driving the expression of the protein of interest. Such a vector may further comprise enhancer regions, and/or expression promoting sequences such as insulators, boundary elements, LCRs (e.g. described by (Blackwood and Kadonaga, 1998) or matrix/scaffold attachment regions (e.g. described by (Li *et al*., 1999). Internal ribosomal entry sites (IRES) may also be present between distinct ORFs present in the Lupac expression vector.

Alternatively, the Lupac expression vector comprises a promoter that drives both the expression of the gene of interest and the expression of Lupac, the ORF of Lupac being separated from the ORF of the protein of interest by the presence of an IRES. The IRES may be derived from, e.g., a virus or a cellular gene.

The term "promoter" as used herein refers to a region of DNA that functions to control the transcription of one or more DNA sequences, and that is structurally identified by the presence of a binding site for DNA-dependent RNA-polymerase and of other DNA sequences, which interact to regulate promoter function. A functional expression promoting fragment of a promoter is a shortened or truncated promoter sequence retaining the activity as a promoter. Promoter activity may be measured in any of the assays known in the art, e.g. in a reporter assay using Luciferase as reporter gene (Wood *et al*., 1984; SELIGER and McELROY, 1960; de Wet *et al.,* 1985), or commercially available from Promega®). An "enhancer region" refers to a region of DNA that functions to increase the transcription of one or more genes. More specifically, the term "enhances", as used herein, is a DNA regulatory element that enhances, augments, improves, or ameliorates expression of a gene irrespective of its location and orientation vis-à-vis the gene to be expressed, and may be enhancing, augmenting, improving, or ameliorating expression of more than one promoter.

In a preferred embodiment, the Lupac expression vector comprises at least one promoter of the murine CMV immediate early region. The promoter may for example be the promoter of the mCMV IE1 gene (the "IE1 promoter"), which is known from, e.g., WO 87/03905. The promoter may also be the promoter of the mCMV IE2 gene (the "IE2 promoter"), the mCMV IE2 gene itself being known from, e.g., Messene *et al.* (1991). The IE2 promoter and the IE2 enhancer regions are described in details in PCT/EP2004/050280. Preferably, the Lupac expression vector comprises at least two promoters of the murine CMV immediate early region. More preferably, the two promoters are the IE1 and the IE2 promoters. Most preferably, the Lupac expression vector comprises SEQ ID NO: 7, which comprises the IE1 promoter, the IE2 promoter and an enhancer region.

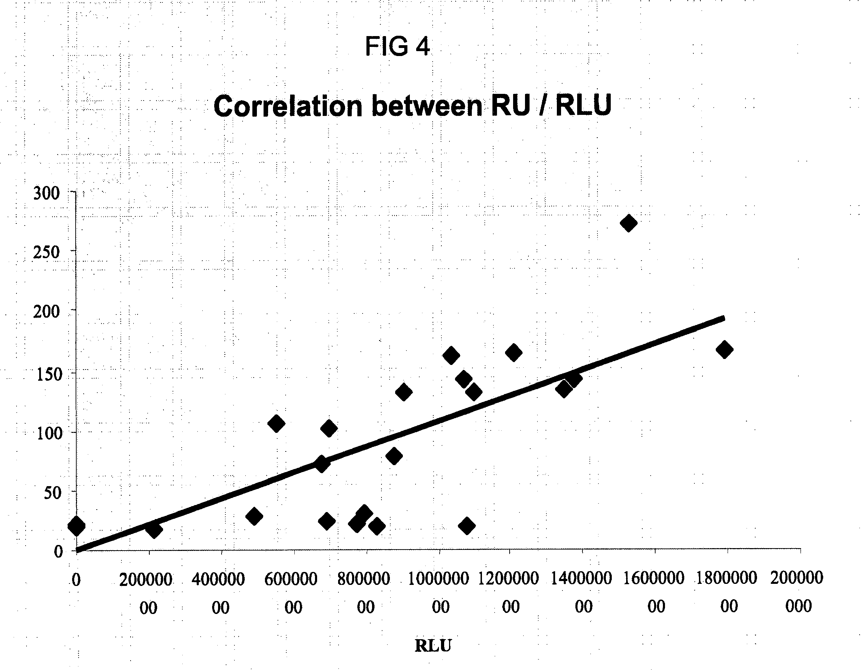

In a preferred embodiment, the Lupac expression vector comprises at least two promoters of the murine CMV immediate early region, wherein one of them drives the expression of a Lupac polypeptide, and the other one drives the expression of a protein of interest. This embodiment is exemplified by the pBSI.IL18BPmCMVLupac.I vector shown on Figure 4, wherein the IE1 promoter drives the expression of a Lupac polypeptide and the IE2 promoter drives the expression of a protein of interest.

In another preferred embodiment, the promoters of the murine CMV immediate early region drive the expression of genes encoding a protein of interest, and the Lupac polypeptide is expressed from an additional expression cassette inserted in the vector backbone. The IE1 and IE2 promoters may drive the expression either of two identical copies of the gene encoding the protein of interest, or of two subunits of a multimeric protein of interest such as antibodies or peptide hormones.

In another preferred embodiment, the Lupac expression vector comprises an amplification marker. This amplification marker may be selected from the group consisting of, e.g., adenosine deaminase (ADA), dihydrofolate reductase (DHFR), multiple drug resistance gene (MDR), ornithine decarboxylase (ODC) and N-(phosphonacetyl) -L-aspartate resistance (CAD). Amplification of the gene encoding the protein of interest allows increasing the expression level of the protein of interest upon integration of the vector in a cell (Kaufman *et al.,* 1985).

A fourth aspect of the invention relates to a cell transfected with a Lupac vector. Many cells are suitable in accordance with the present invention, such as primary or established cell lines from a wide variety of eukaryotes including plant and animal cells. Preferably, said cell is an eukaryotic cell. More preferably, said cell is a mammalian cell. Most preferably, said cell is a Chinese hamster cell or a human cell.

For example, suitable cells include NIH-3T3 cells, COS cells, MRC-5 cells, BHK cells, VERO cells, CHO cells, rCHO-tPA cells, rCHO - Hep B Surface Antigen cells, HEK 293 cells, rHEK 293 cells, rC127 - Hep B Surface Antigen cells, CV1 cells, mouse L cells, HT1080 cells, LM cells, YI cells, NS0 and SP2/0 mouse hybridoma cells and the like, RPMI-8226 cells, Vero cells, WI-38 cells, MRC-5 cells, Normal Human fibroblast cells, Human stroma cells, Human hepatocyte cells, human osteosarcoma cells, Namalwa cells, human retinoblast cells, PER.C6 cells and other immortalized and/or transformed mammalian cells.

### 3. Methods of using Lupac

A fifth aspect relates to the use of a cell comprising a Lupac nucleic acid for producing a protein of interest. Preferably, said cell comprises a Lupac vector.

As discussed in Examples 3.3.2., 3.3.3 and 4, using a Lupac polypeptide as a selection and surrogate marker provides numerous advantages for screening cells for high expression of a protein of interest. Specifically, since the expression of the Lupac polypeptide is highly correlated with the expression of the protein of interest, it is advantageous to perform a primary screen for high Lupac expression. The expression of the protein of interest is assayed in a secondary screen, which is only performed with the best producers isolated further to the primary screen for high Lupac expression.

Accordingly, a sixth aspect of the invention relates to the use of a Lupac polypeptide, of a Lupac nucleic acid or of a Lupac expression vector for screening cells for expression or for high expression of a protein of interest. The cells are first screened for high expression of Lupac, and expression of Lupac is then correlated to that of a protein of interest by inference. This allows to rapidly eliminate 80 to 95% of the tested cells based on low Lupac expression levels, and to retain the remaining 5-20% for analysis of expression of the gene of interest in a second step.

In the context of the uses and methods of the present invention, "high expression" refers to an expression level in a cell that is screened that is higher than in other cells that are screened. "High expression" of a protein is a relative value. For example, final expression levels of recombinant proteins that are commercially produced range from 1 to 2'000 mg.l⁻¹ depending on the protein, annual quantities required and therapeutic dose. During a screening, the expression level of a protein of interest is lower than the final expression level.

A seventh aspect relates to a method of screening cells for expression or high expression of a protein of interest, said method comprising the step of:
(i) transfecting cells by a Lupac expression vector;
(ii) selecting cells being resistant to puromycin; and
(iii) assaying the luciferase activity of the cells selected in step (ii).

In a preferred embodiment, the 20% of cells that exhibit highest luciferase activity in step (iii) comprise the cell that exhibit highest expression of said protein of interest. Preferably, the 10% of cells that exhibit highest luciferase activity in step (iii) comprise the cell that exhibit highest expression of said protein of interest. Most preferably, the 1% or the 5% of cells that exhibit highest luciferase activity in step (iii) comprise the cell that exhibit highest expression of said protein of interest.

Preferably, luciferase activity is measured in step (iii) of the above method by the Bright-Glo luciferase assay on a Centro LB 960 luminometer during 5 seconds acquisition time.

Any number of cells may be screened by such a method. Preferably, the luciferase activity of at least 20, 50, 100, 500, 1'000, 5'000, 10'000, 50'000, 100'000, 500'000 or 1'000'000 cells is assayed in step (iii). Most preferably, a population of cells sufficient for obtaining at least 1'000 to 10'000'000 independant transfectants being resistant to puromycin is screened. Out of these, at least 10 to 1'000'000 candidate clones being resistant to puromycin can further be assayed for luciferase activity.

The cells obtained at the end of the above screening method may be ranked relative to each other regarding Lupac expression. The cells exhibiting the highest luciferase activity may be selected at the end of any of the above methods of screening. For example, individual cells exhibiting luciferase activity corresponding to the top 5-20% of Lupac expressors are selected for further analysis of expression of the gene of interest in a subsequent step.

In a preferred embodiment, the above screening method further comprises the step of (iv) selecting about 1% to about 20% of the cells assayed in step (iii), wherein the selected cells are those exhibiting highest luciferase activity in step (iii). About 5% to about 20% of the cells assayed in step (iii) may be selected based on highest Lupac activity. Alternatively, about 1%, 1,5%, 2%, 3%, 4%, 5% to about 30%, 40%, 50%, 60%, 70% or 80% of the cells assayed in step (iii) may be selected based on highest Lupac activity.

In another preferred embodiment, the above method of screening is performed using multiwell microtiter plates or similar.

Upon selection of the cells exhibiting the highest luciferase activity, the expression level of the protein of interest in said selected cells may further be assayed.

Thus an eight aspect relates to a method of obtaining a cell line expressing a protein of interest, said method comprising the step of:
(i) screening cells according to any of the above methods of screening;
(ii) selecting the cell exhibiting the highest expression of said protein of interest; and
(iii) establishing a cell line from said cell.

As used herein, a "cell line" refers to one specific type of cell that can grow in a laboratory. A cell line can usually be grown in a permanently established cell culture, and will proliferate indefinitely given appropriate fresh medium and space. Methods of establishing cell lines from isolated cells are well-known by those of skill in the art.

A ninth aspect relates to a method of producing a protein of interest, said method comprising the step of:
(i) culturing a cell line obtained as described above under conditions which permit expression of said protein of interest; and
(ii) collecting said protein of interest.

Conditions which permit expression of the protein of interest can easily be established by one of skill in the art by standard methods. For example, the conditions disclosed in Example 3.3.1 may be used.

In a preferred embodiment, the above method of producing a protein of interest further comprises the step of purifying said protein of interest. The purification may be made by any technique well-known by those of skill in the art. In the case of a protein of interest for use in the field of pharmaceutics, the protein of interest is preferably formulated into a pharmaceutical composition.

A tenth aspect relates to a method of producing a Lupac polypeptide comprising the step of:
(i) culturing a cell comprising a Lupac nucleic acid under conditions which permit expression of the Lupac polypeptide; and
(ii) collecting the Lupac polypeptide.

Such a method may for example be performed as described in Example 2. Such a method may further comprise the step of purifying the Lupac polypeptide according to any method known in the art.

Having now fully described this invention, it will be appreciated by those skilled in the art that the same can be performed within a wide range of equivalent parameters, concentrations and conditions without departing from the spirit and scope of the invention and without undue experimentation.

While this invention has been described in connection with specific embodiments thereof, it will be understood that it is capable of further modifications. This application is intended to cover any variations, uses or adaptations of the invention following, in general, the principles of the invention and including such departures from the present disclosure as come within known or customary practice within the art to which the invention pertains and as may be applied to the essential features hereinbefore set forth as follows in the scope of the appended claims.

### EXAMPLES

### Example 1: Construction of Lupac

### 1.1. Obtention of a Lupac nucleic acid by PCR

Lupac was constructed by fusing the open reading frames for firefly luciferase and PAC by PCR cloning.

The template DNA for Luciferase was the pGL3-ctrl plasmid (Promega, cat # E1741) and the template DNA for puromycine acetyl transferase was the pPUR plasmid (Clontech, cat # 6156-1). Two couples of PCR primers (SEQ ID Nos. 3-6), allowing amplification and fusion of the 3' end of luciferase to the 5' end of puromycin acetyl transferase, were designed. The first one amplifies the Luciferase gene from the PpuMI site to the last amino acid excluding the stop codon, and the second one fused the 3' end of Luciferase to the 5'of puromycin acetyl transferase amplification, excluding the initial Methionin acting as translation start codon.

The PCR conditions were as follows:
- *Amplification of Luciferase:* 50 pmol of primers od SEQ ID Nos. 3 and 4, 20ng of pGL3-ctrl, 200 µM each dNTPs, 1x Thermopol Bluffer, 2 units of Vent DNA polymerase (New England Biolabs, cat # M0254S, contains 10x buffer), 5% DMSO. total volume is 50µl.
- *Amplification of pac:* Same chemical condition as for luciferase except 50 pmol of primers of SEQ ID Nos. 5 and 6, and 20 ng pPUR .

### • Cycling:

- Denaturation: 1 cycle, 98°C, 5'
- Hybridation/ Polymerisation : 25 cycles at 98°C for 1' and at 60°C, 1'
- Final polymerization: 1 cycle, 72°C, 10'

A band of 474 base pairs was obtained with the PCR for amplifying luciferase, whereas a band of 618 bp base pairs was obtained with the PCR for amplifying pac. Each PCR reaction was purified using Nucleospin Extract kit from Macherey-Nagel following manufacturer's protocol, cat # 740 590 .50.

A 1 µl of each purified PCR fragment was used for performing another PCR reaction as follows:

50 pmol of primers of SEQ ID Nos. 3 and 6 were used. The same mix as described for the first step PCR was used, except that the Vent DNA polymerase was added after the first denaturation step (Hot Start method). The cycling parameters remained essentially the same, except the Hybridisation/Polymerization temperature was increased to 65 °C.

This PCR reaction allows to fuse the two DNA fragments into one fragment of 1092 base pairs.

The PCR fragment was purified by cutting the band from an agarose gel, and centrifuging it in a Corning filter tip (cat #4823) at 9600 rpm for 10' in an Eppendorf table centrifuge. The eluate was then precipitated by adding 2 volume of Ethanol 100 % and centrifuged full speed.

### 1.2. Cloning of said nucleic acid

### 1.2.1. pBS-Lupac

The pellet was resuspended and treated with T4 polynucleotide kinase, (Stratagene, cat # 600103) according to the manufacurer's protocol. The recipient vector was pBluescript II SK(+) (cat # 212205-01,) cut by EcoRV, followed by Calf Intestine Alkaline Phosphatase treatment (Gibco 18009-027) according to the manufacurer's protocol. The plasmid pBS-Lupac was obtained by conventional ligation reaction and cloning in E. coli. The sequence of the plasmid was then verified by sequencing.

### 1.2.2. pGLupac

The verified pBS-Lupac sequence was digested by PpuMI /XbaI, the 1.1 kb band was purified using Corning tips and ethanol precipitation. The recipient vector was pGL3-ctrl digested by PpuMI/ Xbal. The 4.8kb band was purified by Corning tips elution. The vector obtained after ligation, pGLupac, comprised the Lupac sequence of SEQ ID NO: 1, which codes for the Lupac polypeptide of SEQ ID NO: 2.

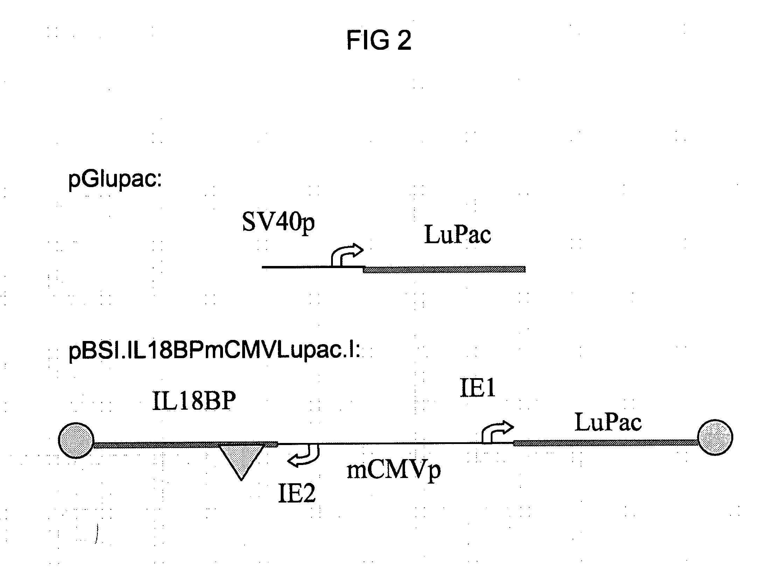

pGlupac is shown on Figure 2. pGlupac contains the ORF for Lupac expressed from a SV40 promoter with an SV40 enhancer at 3'.

### 1.2.3. pGLupac-Basic

pGL3 Basic (Promega, cat # E1751) was cut by NcoI/XbaI. pGLupac is cut by NcoI/Xbal, and the lupac insert of 2.3 kb was purified using Corning tips. The vector obtained after ligation was called pGLupac-Basic.

### 1.2.4. pBSI.IL18BPmCMVLupac.I

pGLupac-Basic was digested by Nhel/Clal/Pvul. The 2.6 kb Nhel/Clal fragment was purified. The recipient vector was obtained by digesting a vector comprising the mouse CMV immediate early region by Nhel and Clal. This recipient vector comprises a DNA sequence of SEQ ID NO: 7 which includes the IE1 and the IE2 promoters as well as the IE1 and IE2 enhancers. A detailed description of the function of a DNA sequence of SEQ ID NO: 7 is provided in PCT application No. PCT/EP2004/050280. The recipient vector further comprised a DNA sequence encoding IL18BP (SwissProt Accession No. 095998).

After ligation, a vector termed pBSI.IL18BPmCMVLupac.I was obtained. pBSI.IL18BPmCMVLupac.I is shown on Figure 2. The expression of IL18BP is driven by the IE2 promoter, whilst the expression of Lupac is driven by the IE1 promoter.

In this construct, IL18BP corresponds to a protein of interest to be produced and Lupac to the surrogate marker.

### Example 2: Characterization of Lupac's function

Assays were performed in order to determine whether Lupac confers a dual function to stably transfected cells, namely measurable luciferase activity and resistance to puromycin. This was tested by transfecting CHO cells with an expression vector for Lupac. As a control, CHO cells were co-transfected with an expression vector for wild type firefly luciferase and an expression vector for pac.

### 2.1. Transfection of CHO cells using Lipofectamine.

The CHO-S cells were purchased from Gibco/ Invitrogen (Cat no:11619).

### Format: 6 well plate

CHO-S in exponential growth phase were diluted to 0.75 x 10⁶ cells/ml twenty-four hours before transfection.

0.6 x 10⁶ cells were resuspended in 440 µl ProCho5 medium (Cambrex, cat #12766Q) supplemented with 4,5 mM Glutamine and 1x Hypoxanthine/Thymidine (HT). (100x HT, Invitrogen, Cat.#:11067-030, L-Glutamine 200 mM, Sigma, G-7513)

Two mixes were prepared:
• Mix A: Lipofectamine (Invitrogen, Cat No:18324-012): 8.8 µl
   ProCho5 Medium:211.2 µl
   Total volume: 220 µl.
• Mix B: 1 µg plasmid DNA for either control or lupac construct, respectively.
   *Control:* 0.5 µg of pGL3-Ctrl + 0.5 µg of pPur.
   *Lupac:* 0.5 µg pGLupac+ 0.5 µg of an irrelevant plasmid (pBluescript II SK(+)).
   ProCho5 Medium : complement to 220 µl.
Mixes A and B were mixed together, and let at room temp for 30 min.

This A+B mix was added to the 440 µl of medium containing 0.6 x 10⁶ cells. The cell suspension was placed in an incubator, 37°C, 5% CO₂ for 3 hours, and 1.6 ml ProCho5 supplemented with 1X HT and 4,5 mM L-Glutamine was then added. The cell suspension was further incubated under the same conditions.

### 2.2. Luciferase Measurement:

### 2.2.1. Protocol

Luciferase activity was measured two days after transfection. The Bright-Glo Luciferase assay system was purchased from Promega (Cat No : E2610). The assay was performed according to the manufacturer's guidelines. Briefly, the cell suspension was homogenized by pipetting up and down several times, and an aliquot of 50 µl was taken out and put it in a white 96 well plate (Nunc, Cat no:236108). 50 µl of reconstituted Bright-Glo Reagent was added directly, and the cell suspension was incubated for 5 min at room temp. Light emission was measured on a Centro LB 960 luminometer (Berthold Technologies) and acquisition time was 5 sec. Light emission is measured in Relative Light Units (RLU). The results were normalized for cell number (by cell density in million /ml).

### 2.2.2. Results

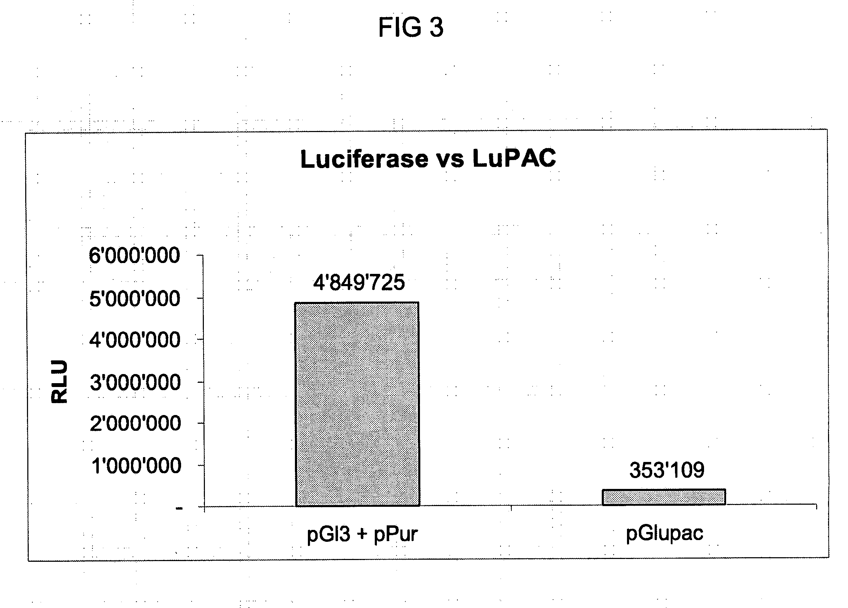

The results are shown on Figure 3. Luciferase activity was measured both for cells transfected with pGlupac and for cells transfected with pGI3-ctrl+pPur. Accordingly, Lupac exhibits luciferase activity.

### 2.3. Selection for resistance to puromycin

### 2.3.1. Protocol

After Luc measurement, the remaining cells from the 6 well plate were transferred to a 15 ml Falcon tube, centrifuged, and the cell pellet was resuspended in 2 ml medium containing 5% Fetal Bovine Serum (FBS) in a 6 well plate. Selection was applied 48 hours post transfection, by exchanging the medium for ProCho5/HT/Glutamine/5% FBS containing 10 µg/ml of puromycin (Sigma, P-8833). Every two days, a medium exchange was performed by discarding the old medium, washing with 1x PBS, and adding fresh selective medium. After 2 weeks of selection, the cells were trypsinised, counted, and a series of dilutions corresponding to 1000, 500, 100, 50, 20, 10 cells/well of a 6-w format was performed. Ten days later, the colonies growing in all dilutions were counted, and all of them were picked to allow growth in suspension in the absence of serum for protoclone analysis.

For the positive control co-transfection with pGL3-Ctrl and pPur, fifty seven (57) clones grew.

For the negative control, corresponding to untransfected CHO-S wild-type cells, no clones grew.

For the transfection with pGlupac and pBluescript II SK(+), forty eight (48) clones grew.

Thus a similar amount of clones growing in the selective medium was observed. Accordingly, it can be concluded that the puromycin resistance conferred by the fusion protein is comparable to the puromycin resistance conferred by the wild-type puromycin resistance gene.

The luciferase activity of the resistant clones was then measured. Luciferase measurement identified higher percentage of clones expressing both functions upon transfection with pGLupac than upon co-transfection with luciferase and the puromycin resistance gene on separate vectors (Table IV). This confirms the efficacy of Lupac.

**Table IV**

| | Number of puromycin-resistant clones analyzed | % of clones expressing Luciferase |
|---|---|---|
| pGL3-ctrl + SVp-Puro | 57 | 82 |
| pGLupac | 48 | 98 |

In conclusion, the Lupac fusion protein shows the combined activity and function of both luciferase and pac proteins.

### Example 3: Use of Lupac as a surrogate marker

The dual function of the created Lupac fusion protein suggests that it should also have a dual impact. First, Lupac should allow the isolation of stably transfected clones by their resistance to puromycin, and secondly, Lupac expression levels should reflect expression levels of a physically connected gene of interest by measurement of luciferase activity. In order to test this hypothesis, a series of clones from pools of cells stably transfected with pBSI.IL18BPmCMVLupac.I were generated. Lupac activity and IL 18BP expression levels were measured.

### 3.1. Transfection of CHO cells using Lipofectamine.

The CHO-S cells were purchased from Gibco/ Invitrogen (Cat no:11619).

### Format: T75 flasks.

CHO-S cells in exponential growth phase were passaged 24 h before transfection. They were diluted to 0.75 x 10⁶ cells/ml.

5 x 10⁶ cells were resuspended in 7 ml ProCho5 medium ( Cambrex Cat no:12766Q) supplemented with 1X HT (Invitrogen, Cat.no:11067-030) and 4,5 mM L-Glutamine (Sigma, Cat.no:G-7513) in a T75 flask.

Two mixes were prepared:
• Mix A: Lipofectamine (Invitrogen, Cat No:18324-012): 52,1 µl
   ProCho5 Medium: 517,9 µl
   Total volume: 570 µl.
• Mix B: DNA: 10 µg of total DNA containing 5 µg of pBSI.IL18BPmCMVLupac.I, linearized with Xmnl, and 5 µg of an irrelevant plasmid (pBluescript II SK+)
   ProCho5 Medium : complement to 570 µl.
Mixes A and B were combined, and incubated for 30 min at room temperature.

This A+B mix was added to the 7 ml containing 5 x 10⁶ cells. This suspension was placed back in an incubator at 37°C, 5% CO₂ for 3 hours. The culture was then centrifuged at 800g for 3 minutes, and the cell pellet resuspended in 5 ml ProCho5 supplemented with 1X HT and 4,5 mM L-Glutamine. 5 ml of ProCho5/HT/Glutamine were then added directly to the T75 flask, and added to the suspension. At the end, 5 x 10⁶ cells were in 10 ml ProCho5/ HT/ Glutamine medium.

### 3.2. Selection Procedure

The selection was applied 48 hours post transfection, by exchanging the medium and diluting to 0.5 x 10⁶ cells / ml in ProCho5 / HT/ Glutamine containing 10 µg/ ml of puromycine (Sigma, P-8833). Every two days, cells were counted, centrifuged, and resuspended in fresh selective medium at 0.5 x 10⁶ living cells / ml. Viability is checked at these points. After 21 to 35 days the selection was completed. The viability, after the expected initial drop, reached more than 80 % again.

Once the stable pool established, cells were seeded in 384 well plates (Nunc, cat. No.: 164688) at a density of 1 cell per well (70 µl/well) using a Multidrop dispenser (ThermoLabsystems, cat. 5840150), and randomly picked clones were re-arrayed in one 96 well plate two weeks later.

### 3.3. Analysis of expression

### 3.3.1. Protocol

In order to evaluate expression of both genes a high throughput format was used (96 well plates).
- Day 1: A 50% dilution of the cells was performed in 100 µl of ProCho5 culture medium (serum free) + 100 µl of fresh ProCho5 containing 5 % Fetal Bovine Serum. Weekly passage of the maintenance plate was done in a 1/20 dilution factor in ProCho5 medium devoid of serum.
- Day 2: Medium was discarded, washed once with 200 µl 1x PBS (Invitrogen, Cat. No.: 10010-015). 75 µl fresh ProCho5 containing 5% FBS was added, and the suspension was incubated for a 24 h expression pulse.
- Day 3: 50 µl of the supernatants were recovered, and 200 µl Elisa buffer added (1x PBS, 0.1 % w/v BSA, 0.2 % v/v Tween 20). 100 µl were analyzed by a standard ELISA assay for IL18BP.

The wells were washed with 200 µl 1x PBS (discard) and 100 µl Glo Lysis buffer (Promega, E266a) were added. The wells were incubated for 30 min at room temperature to ensure cell lysis.

Luciferase measurement was done using 30 µl lysed cells transferred in a white 96 well plate (Nunc Cat 236108) + 30 µl reconstituted Bright-Glo reagent (Promega, E263a). Light emission was measured on a Centro LB960 luminometer during 5 seconds acquisition time.

In Figure 4, IL18BP expression level is measured by a Biacore instrument in Response Units (RU, a measure given by the manufacturer) and luciferase activity is measured in Relative Light Units (RLU).

### 3.3.2. Results

Eighty-five (85) candidate clones were randomly selected and assayed for both Lupac and IL18BP expression. Twenty-four (24) individual clones where selected for further studies: 8 clones each representing high, medium or low Lupac expression respectively. All 24 clones were then re-assayed for expression of both genes.

Figure 4 shows the correlation observed with these experiments. There is a very good correlation between Lupac and IL18BP expression, especially at both ends of the range. In other words, the highest expressors for Lupac also correspond to the highest expressors for IL188P.

Lupac can thus be used as a selective and surrogate marker to establish and screen candidate clones with a vector expressing both Lupac and the protein of interest. For example, a primary screen can be done for high Lupac expression with a high probability of selecting clones that also exhibit high gene of interest expression. Thus, very tedious, lengthy and costly screens using ELISA or other approaches can be avoided. Furthermore, screening for Lupac is independent from the specific gene of interest that is chosen, so the same approach can be used for a variety of screening programs, which is a clear logistical advantage. Gene of interest expression will only be assayed in a secondary screen of the best producers from the primary screen.

### 3.3.3. Conclusion

Using Lupac in HTS will allow keeping the same chance for selecting high expressing clones, and allow reducing time and resources. In a classical HTS done generation approach, the best clones are typically chosen on the basis of high titers for secreted proteins upon screening of more than 2,000 clones. Using Lupac, a done giving a similar productivity for IL18BP was obtained upon screening of only 85 clones. The reduction of the screening sample size to get similar productivities of a gene of interest may relate to the ease of use of the Lupac approach and the associated reduction of sampling errors and assay variance related to ELISA high throughput screens. In addition, by selecting the 5 to 10 best clones per plate, the best clone per plate is expected to be selected. Thus, using Lupac for screening 1,000 clones will reduce the number of clones to be analyzed to 50 to 100, and thus allow the avoidance of a second HTS.

In addition, it is important to note that the fusion of two individual enzymes with so different activities and origins surprisingly retains their function in Lupac as it is described here. The retained dual function clearly leads to a dual impact as Lupac can truly be used to provide selectivity in stable transfection and act as a surrogate marker for screening candidate clones for high expression of a gene of interest.

Finally, it is also worth to note that in the present experiments, the expression of intracellular Lupac is highly correlated with the expression of the secreted IL18BP. Other high throughput techniques, such as cell sorting with a FACS, clearly have their limitations when screening for secreted proteins.

### Example 4: Obtention of a high producer clone for r-SP1

The aim of this experiment was to develop a high producer CHO cell line for a recombinant protein referred to as recombinant Serono Protein 1 (r-SP1).

No direct high throughput screening method for the r-SP1 protein did exist. The commercially available Elisa assay for measuring the quantity of r-SP1 in a sample only allowed analyzing eight (8) samples per microtiter plaque due to its low sensitivity. Such an Elisa assay is qualified as a "low throughput" Elisa assay to the contrary to "high throughput" Elisa assays that allow analyzing close to ninety-six (96) samples per microtiter plaque.

It would be an extremely tedious and time-consuming process to screen for high producer clones for r-SP1 using the commercially available low throughput Elisa assay (if at all realistic). Instead of using such a lengthy process, it will be shown below that high producer clones were rapidly and successfully screened using Lupac as a surrogate marker.

### 4.1. Experimental approach for the screening

A flowchart of the experimental approach is shown in Table V.

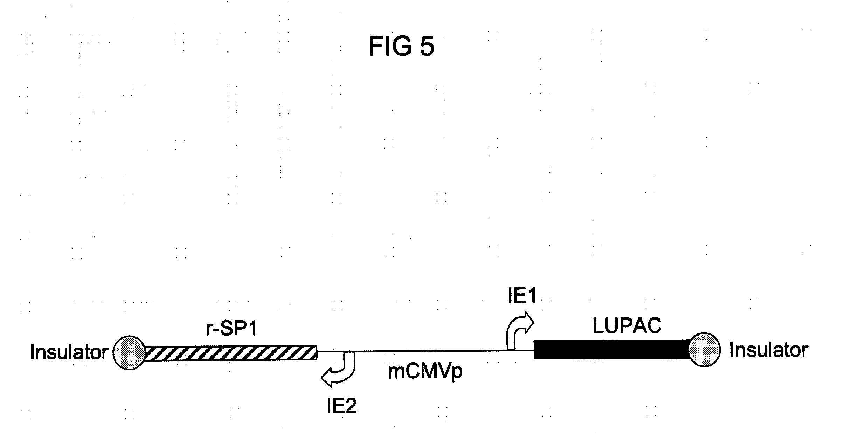

The expression vector coding for the recombinant protein r-SP1 and combining the bi-directional mouse CMV IE1 and IE2 promoters with other elements necessary for efficient expression is shown in Figure 5. This vector co-expresses r-SP1 and Lupac. The pools of stably transfected cells were established in the presence of puromycin. Following selection, titer and specific productivity (pcd) of r-SP1 were evaluated at the level of the pools, and the two best pools were subjected to clone isolation. 700 clones for each selected pool were analysed in two rounds of high throughput luciferase assay. The clones expressing the highest levels of Lupac were selected. This allowed reducing the number of clones from 1400 to 19 in only two days.

These 19 clones were further analyzed in quantitative ELISA for titer and specific productivity of r-SP1 in order to select the clones expressing the highest levels of r-SP1. These clones underwent a further round of cloning in order to ensure the obtention of independent clones. Specific productivity (pcd) was analyzed for the independent clones. A Master Cell Bank was established for the most promising clone. The resulting cell line was referred to as CHO-r-SP1.

**Table V**

| |
|---|
| **Construction of the expression vector** |
| ↓ |
| **Transfection of the expression vector into CHO cells** |
| • suspension culture in serum-free medium |
| ↓ |
| **Establishment of stable pools:** |
| • selection in the presence of puromycin |
| ↓ |
| **Isolation of 1400 candidate clones:** |
| • limiting dilution at 1 cell/well in 384 well plates |
| • re-arraying of candidate clones from 384 into 96 well plates |
| ↓ |
| **Screening in 96 well plates using a high throughput luciferase ELISA assay:** |
| • 1400 candidate clones → 19 candidate clones in only two days |
| ↓ |
| **Measurement of r-SP1 expression using a low throughput r-SP1 ELISA assay:** |
| • 19 candidate clones → 5 candidate clones |
| ↓ |
| **Re-cloning of the 5 candidate clones** |
| ↓ |
| **Clone evaluation** |
| ↓ |
| **Selection of the best producer clone** |
| ↓ |
| **Construction of a Master Cell Bank** |
| ↓ |
| **Process development and production of r-SP1** |

### 4.2. Production of r-SP1 from the CHO-r-SP1 cell line

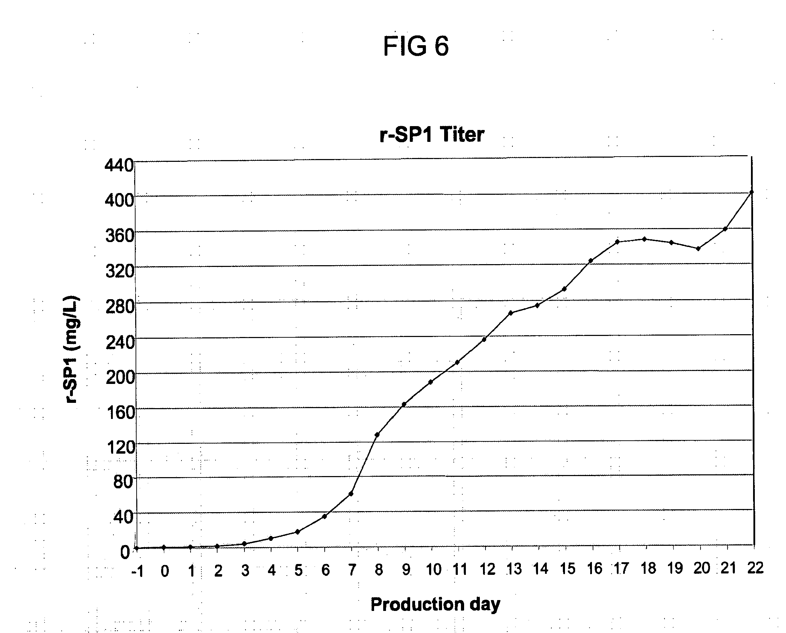

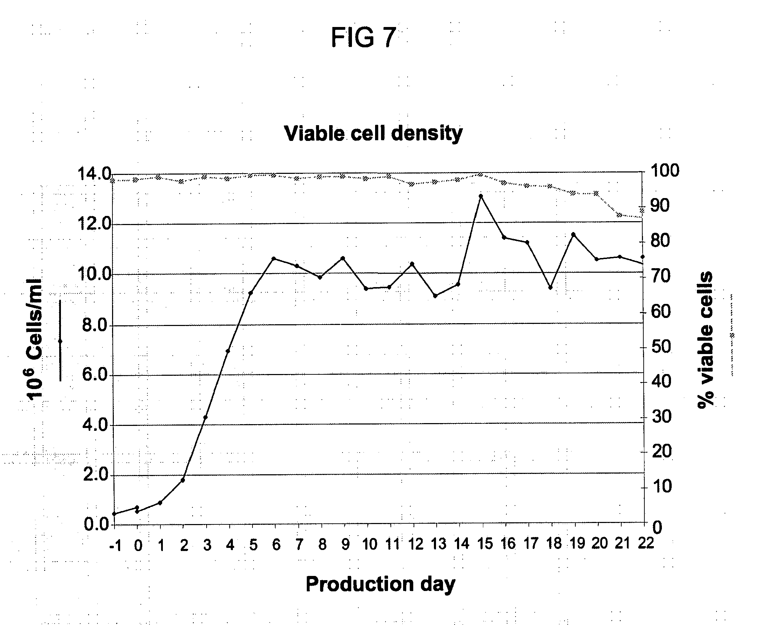

The CHO-r-SP1 cell line was cultivated in suspension in a serum-free medium in a 250 L bioreactor using a fed-batch process. As shown on Figures 6 and 7, the r-SP1 titer reached levels as high as 401 mg/L after 22 days. The cell viability was still very good at the end of the run. The CHO-r-SP1 cell line growed to high cell density (10 millions/ml) with an average specific productivity of 2 pcd.

### 4.3. Conclusion

This experiment validates the use of Lupac in a real screening format. The use of Lupac as a surrogate marker allowed successfully selecting a producer clone that expressed high levels of a protein of interest for which no direct high throughput screening method was available.

### REFERENCES

Altschul,S.F., Gish,W., Miller,W., Myers,E.W., and Lipman,D.J. (1990). Basic local alignment search tool. J. Mol. Biol. 215, 403-410.
Bennett,R.P., Cox,C.A., and Hoeffler,J.P. (1998). Fusion of green fluorescent protein with the Zeocin-resistance marker allows visual screening and drug selection of transfected eukaryotic cells. Biotechniques 24, 478-482.
Blackwood,E.M. and Kadonaga,J.T. (1998). Going the distance: a current view of enhancer action. Science 281, 61-63.
Borth,N., Zeyda,M., Kunert,R., and Katinger,H. (2000). Efficient selection of high-producing subclones during gene amplification of recombinant Chinese hamster ovary cells by flow cytometry and cell sorting. Biotechnol. Bioeng. 71, 266-273.
Chesnut,J.D., Baytan,A.R., RusseII,M., Chang,M.P., Bernard,A., Maxwell,I.H., and Hoeffler,J.P. (1996). Selective isolation of transiently transfected cells from a mammalian cell population with vectors expressing a membrane anchored single-chain antibody. J. Immunol. Methods 193, 17-27.
de Wet,J.R., Wood,K.V., Helinski,D.R., and DeLuca,M. (1985). Cloning of firefly luciferase cDNA and the expression of active luciferase in Escherichia coli. Proc Natl Acad Sci U S A 82, 7870-7873.
Devereux,J., Haeberli,P., and Smithies,O. (1984). A comprehensive set of sequence analysis programs for the VAX. Nucleic Acids Res. 12, 387-395.
Grantham,R. (1974). Amino acid difference formula to help explain protein evolution. Science 185, 862-864.
Kaufman,R.J., Wasley,L.C., Spiliotes,A.J., Gossels,S.D., Latt,S.A., Larsen,G.R., and Kay,R.M. (1985). Coamplification and coexpression of human tissue-type plasminogen activator and murine dihydrofolate reductase sequences in Chinese hamster ovary cells. Mol. Cell Biol. 5, 1750-1759.
Li,Q., Harju,S., and Peterson,K.R. (1999). Locus control regions: coming of age at a decade plus. Trends Genet. 15, 403-408.
Messerie,M., Keil,G.M., and Koszinowski,U.H. (1991). Structure and expression of murine cytomegalovirus immediate-early gene 2. J. Virol. 65, 1638-1643.
Pearson,W.R. (1990). Rapid and sensitive sequence comparison with FASTP and FASTA. Methods Enzymol. 183, 63-98.
Pearson,W.R. and Lipman,D.J. (1988). Improved tools for biological sequence comparison. Proc. Natl. Acad. Sci. U. S. A 85, 2444-2448.
SELIGER,H.H. and McELROY,W.D. (1960). Spectral emission and quantum yield of firefly bioluminescence. Arch. Biochem. Biophys. 88, 136-141.
Smith,T.F. and Waterman,M.S. (1981). Identification of common molecular subsequences. J. Mol. Biol. 147, 195-197.
Wood,K.V., de Wet,J.R., Dewji,N., and DeLuca,M. (1984). Synthesis of active firefly luciferase by in vitro translation of RNA obtained from adult lanterns. Biochem Biophys. Res. Commun. 124, 592-596.

## Claims

1. A Lupac polypeptide comprising a fragment of a luciferase fused to a fragment of a puromycin N-acetyl transferase (pac), wherein:
a) said Lupac polypeptide exhibits:
(i) luciferase activity; and
(ii) puromycin N-acetyl transferase activity.
b) said fragment of a luciferase comprises an amino acid sequence at least 95% identical to a *Photinus pyralis* luciferase of SEQ ID NO: 8, and
c) said fragment of a pac comprises an amino acid sequence at least 95% identical to a *Streptomyces alboniger* pac of SEQ ID NO: 9.

2. The Lupac polypeptide of claim 1, wherein said fragment of a pac comprises amino acids 2 to 199 of SEQ ID NO: 9.

3. The Lupac polypeptide of claim 1 or 2, wherein said fragment of a luciferase is fused to the 5' terminus of said fragment of a pac.

4. The Lupac polypeptide of claim 1 or 2, wherein said fragment of a pac is fused to the 5' terminus of said fragment of a luciferase.

5. The Lupac polypeptide of claim 3, wherein said Lupac polypeptide comprises an amino acid sequence at least 95% identical to SEQ ID NO: 2.

6. The Lupac polypeptide of claim 5, wherein said Lupac polypeptide comprises SEQ ID NO: 2.

7. A nucleic acid encoding the Lupac polypeptide of any of claims 1 to 6.

8. The nucleic acid of claim 7, wherein said nucleic acid comprises SEQ ID NO: 1.

9. A vector comprising the nucleic acid of claim 7 or 8.

10. The vector of claim 9, wherein said vector is an expression vector.

11. The expression vector of claim 10, wherein said vector further comprises a nucleic acid encoding a protein of interest.

12. The expression vector of claim 11, wherein said vector comprises at least two promoters, one driving the expression of said Lupac polypeptide, and the other one driving the expression of said protein of interest.

13. The expression vector of claim 12, wherein said at least two promoters are promoters of the murine CMV immediate early region.

14. The expression vector of claim 13, wherein said at least two promoters are the IE1 and the IE2 promoters.

15. The expression vector of any of claims 11 to 14, wherein said vector further comprises an amplification marker selected from the group consisting of adenosine deaminase (ADA), dihydrofolate reductase (DHFR), multiple drug resistance gene (MDR), ornithine decarboxylase (ODC) and N-(phosphonacetyl) -L-aspartate resistance (CAD).

16. A cell comprising the nucleic acid of claim 7 or 8.

17. The cell of claim 16, wherein said cell comprises the vector of any of claim 9 to 15.

18. The cell of claim 16 or 17, wherein said cell is a mammalian cell.

19. The cell of claim 18, wherein said cell is a CHO cell.

20. The cell of claim 18, wherein said cell is a human cell.

21. Use of the cell of any of claims 16 to 20 for producing a protein of interest.

22. An in vitro use of the vector according to any of claims 11 to 15 for screening cells for expression of a protein of interest.

23. Use of claim 22 wherein said expression of a protein of interest is correlated to luciferase activity.

24. An in vitro method of screening cells for expression of a protein of interest, said method comprising the step of:
(i) transfecting cells by a Lupac expression vector according to claim 10;
(ii) selecting cells being resistant to puromycin; and
(iii) assaying the luciferase activity of the cells selected in step (ii).

25. The method of claim 24, wherein said expression vector is the expression vector of any of claims 11 to 15.

26. The method of claim 24 or 25, wherein the 5%, 10%, 15% or 20% of cells that exhibit highest luciferase activity in step (iii) comprise the cell that exhibit highest expression of said protein of interest.

27. The method of any of claims 24 to 26, further comprising the step of:
(iv) selecting about 1% to about 20% of the cells assayed in step (iii), wherein the selected cells are those exhibiting highest luciferase activity in step (iii).

28. The method of claim 27, further comprising the step of:
(v) assaying the expression level of the protein of interest in the cells selected at the end of step (iv).

29. The method of claim 28, further comprising the steps of:
(vi) selecting the cell exhibiting the highest expression of said protein of interest; and
(vii) establishing a cell line from said cell.

30. The method of claim 29, further comprising the steps of:
(viii) culturing a cell line obtained according to the method of claim 29 under conditions which permit expression of said protein of interest; and
(ix) collecting said protein of interest.

31. The method of claim 30, further comprising the step of purifying said protein of interest.

32. The method of claim 31, further comprising the step of formulating said protein of interest into a pharmaceutical composition.

## Patentansprüche

1. Lupac-Polypeptid, umfassend ein Fragment einer Luziferase, welche an ein Fragment einer Puromycin N-Acetyltransferase (pac) fusioniert ist, wobei:
a) das Lupac-Polypeptid aufweist:
(i) Luziferase-Aktivität; und
(ii) Puromycin N-Acetyltransferase-Aktivität.
b) das Fragment einer Luziferase eine Aminosäuresequenz umfasst, die mindestens 95% identisch zu einer *Photinus pyralis* Luziferase aus SEQ ID NR: 8 ist und
c) das Fragment einer pac eine Aminosäuresequenz umfasst, die mindestens 95% identisch zu einer *Streptomyces alboniger* pac aus SEQ ID NR: 9 ist.

2. Lupac-Polypeptid nach Anspruch 1, wobei das Fragment einer pac Aminosäuren 2 bis 199 von SEQ ID NR: 9 umfasst.

3. Lupac-Polypeptid nach Anspruch 1 oder 2, wobei das Fragment einer Luziferase an das 5'-Ende des Fragments von einer pac fusioniert ist.

4. Lupac-Polypeptid nach Anspruch 1 oder 2, wobei das Fragment einer pac an das 5'-Ende des Fragments von einer Luziferase fusioniert ist.

5. Lupac-Polypeptid nach Anspruch 3, wobei das Lupac-Polypeptid eine Aminosäuresequenz umfasst, die mindestens 95% identisch zu SEQ ID NR: 2 ist.

6. Lupac-Polypeptid nach Anspruch 5, wobei das Lupac-Polypeptid SEQ ID NR: 2 umfasst.

7. Nukleinsäure, welche das Lupac-Polypeptid nach einem der Ansprüche 1 bis 6 kodiert.

8. Nukleinsäure nach Anspruch 7, wobei die Nukleinsäure SEQ ID NR: 1 umfasst.

9. Vektor, welcher die Nukleinsäure nach Anspruch 7 oder 8 umfasst.

10. Vektor nach Anspruch 9, wobei der Vektor ein Expressionsvektor ist.

11. Expressionsvektor nach Anspruch 10, wobei der Vektor zusätzlich eine Nukleinsäure umfasst, die ein Protein von Interesse kodiert.

12. Expressionsvektor nach Anspruch 11, wobei der Vektor mindestens zwei Promotoren umfasst, wobei einer die Expression des Lupac-Polypeptids steuert und der andere die Expression des Proteins von Interesse steuert.

13. Expressionsvektor nach Anspruch 12, wobei die mindestens zwei Promotoren Promotoren der murinen CMV unmittelbaren frühen Region sind.

14. Expressionsvektor nach Anspruch 13, wobei die mindestens zwei Promotoren die IE1 und IE2 Promotoren sind.

15. Expressionsvektor nach einem der Ansprüche 11 bis 14, wobei der Vektor zusätzlich einen Amplifikationsmarker ausgewählt aus der Gruppe bestehend aus Adenosindeaminase (ADA), Dihydrofolatreduktase (DHFR), Multiples Arzneimittel-Resistenzgen (MDR), Ornithindecarboxylase (ODC) und N-(phosphonacetyl)-L-Aspartat-Resistenz (CAD) umfasst.

16. Zelle, umfassend die Nukleinsäure nach Anspruch 7 oder 8.

17. Zelle nach Anspruch 16, wobei die Zelle den Vektor nach einem der Ansprüche 9 bis 15 umfasst.

18. Zelle nach Anspruch 16 oder 17, wobei die Zelle eine Säugetierzelle ist.

19. Zelle nach Anspruch 18, wobei die Zelle eine CHO-Zelle ist.

20. Zelle nach Anspruch 18, wobei die Zelle eine humane Zelle ist.

21. Verwendung der Zelle nach einem der Ansprüche 16 bis 20 zur Herstellung eines Proteins von Interesse.

22. In vitro Verwendung des Vektors gemäß einem der Ansprüche 11 bis 15 zum Screenen von Zellen auf die Expression eines Proteins von Interesse.

23. Verwendung nach Anspruch 22, wobei die Expression eines Proteins von Interesse mit Luziferase-Aktivität korreliert ist.

24. In vitro Verfahren zum Screenen von Zellen auf die Expression eines Proteins von Interesse, wobei das Verfahren die Schritte umfasst:
(i) Transfizieren von Zellen mit einem Lupac-Expressionsvektor gemäß Anspruch 10;
(ii) Auswählen von Zellen, welche resistent gegen Puromycin sind und
(iii) Untersuchen der Luziferase-Aktivität der in Schritt (ii) ausgewählten Zellen.

25. Verfahren nach Anspruch 24, wobei der Expressionsvektor der Expressionsvektor nach einem der Ansprüche 11 bis 15 ist.

26. Verfahren nach Anspruch 24 oder 25, wobei die 5%, 10%, 15% oder 20% der Zellen, die höchste Luziferase-Aktivität in Schritt (iii) aufweisen, die Zelle umfassen, die die höchste Expression des Proteins von Interesse aufweist.

27. Verfahren nach einem der Ansprüche 24 bis 26, zusätzlich umfassend den Schritt:
(iv) Auswählen von etwa 1 % bis etwa 20% der Zellen, die in Schritt (iii) untersucht wurden, wobei die ausgewählten Zellen die sind, die die höchste Luziferase-Aktivität in Schritt (iii) aufweisen.

28. Verfahren nach Anspruch 27, zusätzlich umfassend den Schritt:
(v) Untersuchen des Expressionslevels des Proteins von Interesse in den Zellen, die am Ende des Schrittes (iv) ausgewählt wurden.

29. Verfahren nach Anspruch 28, zusätzlich umfassend die Schritte:
(vi) Auswählen der Zelle, die die höchste Expression des Proteins von Interesse aufweist; und
(vii) Etablieren einer Zelllinie von dieser Zelle.

30. Verfahren nach Anspruch 29, zusätzlich umfassend die Schritte:
(viii) Kultivieren einer Zelllinie, welche gemäß dem Verfahren nach Anspruch 29 erhalten wurde, unter Bedingungen, welche die Expression des Proteins von Interesse erlauben; und
(ix) Sammeln des Proteins von Interesse.

31. Verfahren nach Anspruch 30, zusätzlich umfassend den Schritt der Aufreinigung des Proteins von Interesse.

32. Verfahren nach Anspruch 31, zusätzlich umfassend den Schritt der Formulierung des Proteins von Interesse in eine pharmazeutische Zusammensetzung.

## Revendications

1. Polypeptide LuPac, comprenant un fragment d'une luciférase fusionné avec un fragment d'une puromycine N-acétyl-transférase (enzyme Pac),
a) lequel polypeptide LuPac est doté
i) d'une activité de luciférase
ii) et d'une activité de puromycine N-acétyl-transférase ;
et dans lequel polypeptide LuPac
b) ledit fragment d'une luciférase comprend une séquence d'acides aminés qui est identique, à un degré d'au moins 95 %, à celle d'une luciférase de *Photinus pyralis,* donnée en tant que Séquence N° 8 ;
c) et ledit fragment d'une enzyme Pac comprend une séquence d'acides aminés qui est identique, à un degré d'au moins 95 %, à celle d'une enzyme Pac de *Streptomyces alboniger,* donnée en tant que Séquence N° 9.

2. Polypeptide LuPac conforme à la revendication 1, dans lequel ledit fragment d'une enzyme Pac comprend les acides aminés n° 2 à 199 de la Séquence N° 9.

3. Polypeptide LuPac conforme à la revendication 1 ou 2, dans lequel ledit fragment d'une luciférase est fusionné à l'extrémité 5' dudit fragment d'une enzyme Pac.

4. Polypeptide LuPac conforme à la revendication 1 ou 2, dans lequel ledit fragment d'une enzyme Pac est fusionné à l'extrémité 5' dudit fragment d'une luciférase.

5. Polypeptide LuPac conforme à la revendication 3, lequel polypeptide LuPac comprend une séquence d'acides aminés qui est identique, à un degré d'au moins 95 %, à la Séquence N° 2.

6. Polypeptide LuPac conforme à la revendication 5, lequel polypeptide LuPac comprend la Séquence N° 2.

7. Acide nucléique codant un polypeptide LuPac conforme à l'une des revendications 1 à 6.

8. Acide nucléique conforme à la revendication 7, lequel acide nucléique comprend la Séquence N° 1.

9. Vecteur comprenant un acide nucléique conforme à la revendication 7 ou 8.

10. Vecteur conforme à la revendication 9, lequel vecteur est un vecteur d'expression.

11. Vecteur d'expression conforme à la revendication 10, lequel vecteur comprend en outre un acide nucléique codant une protéine intéressante.

12. Vecteur d'expression conforme à la revendication 11, lequel vecteur comprend au moins deux promoteurs dont l'un commande l'expression dudit polypeptide LuPac et l'autre commande l'expression de ladite protéine intéressante.

13. Vecteur d'expression conforme à la revendication 12, dans lequel lesdits promoteurs au nombre d'au moins deux sont des promoteurs de la région précoce immédiate du virus CMV murin.

14. Vecteur d'expression conforme à la revendication 13, dans lequel lesdits promoteurs au nombre d'au moins deux sont les promoteurs IE1 et IE2.

15. Vecteur d'expression conforme à l'une des revendications 11 à 14, lequel vecteur comprend en outre un marqueur d'amplification choisi dans l'ensemble constitué par les marqueurs ADA (adénosine désaminase), DHFR (dihydrofolate réductase), MDR (gène de résistance à de multiples médicaments), ODC (ornithine décarboxylase) et CAD (résistance au N-(phosphonoacétyl)-L-aspartate).

16. Cellule comprenant un acide nucléique conforme à la revendication 7 ou 8.

17. Cellule conforme à la revendication 16, laquelle cellule comprend un vecteur conforme à l'une des revendications 9 à 15.

18. Cellule conforme à la revendication 16 ou 17, laquelle cellule est une cellule mammalienne.

19. Cellule conforme à la revendication 18, laquelle cellule est une cellule CHO.

20. Cellule conforme à la revendication 18, laquelle cellule est une cellule humaine.

21. Utilisation d'une cellule conforme à l'une des revendications 16 à 20 pour la production d'une protéine intéressante.

22. Utilisation *in vitro* d'un vecteur conforme à l'une des revendications 11 à 15 pour la recherche par criblage, chez des cellules, de l'expression d'une protéine intéressante.

23. Utilisation conforme à la revendication 22, dans laquelle ladite expression d'une protéine intéressante est corrélée avec l'activité de luciférase.

24. Procédé *in vitro* de recherche par criblage, chez des cellules, de l'expression d'une protéine intéressante, lequel procédé comporte les étapes suivantes :
i) transfecter des cellules avec un vecteur d'expression de LuPac, conforme à la revendication 10 ;
ii) sélectionner les cellules qui sont résistantes à la puromycine ;
iii) et doser l'activité de luciférase des cellules sélectionnées dans l'étape (ii).

25. Procédé conforme à la revendication 24, dans lequel ledit vecteur d'expression est un vecteur d'expression conforme à l'une des revendications 11 à 15.

26. Procédé conforme à la revendication 24 ou 25, dans lequel la fraction des 5 %, des 10 %, des 15 % ou des 20 % de cellules qui présentent l'activité de luciférase la plus élevée observée au cours de l'étape (iii) comprend les cellules qui présentent le niveau le plus élevé d'expression de ladite protéine intéressante.

27. Procédé conforme à l'une des revendications 24 à 26, qui comporte en outre l'étape suivante :
iv) sélectionner d'environ 1 % à environ 20 % des cellules testées dans l'étape (iii), étant entendu que les cellules sélectionnées sont celles qui présentent l'activité de luciférase la plus élevée observée au cours de l'étape (iii).

28. Procédé conforme à la revendication 27, qui comporte en outre l'étape suivante :
v) déterminer le niveau d'expression de la protéine intéressante chez les cellules sélectionnées à la fin de l'étape (iv).

29. Procédé conforme à la revendication 28, qui comporte en outre les étapes suivantes :
vi) sélectionner une cellule chez laquelle le niveau d'expression de ladite protéine intéressante est le plus élevé ;
vii) et établir une lignée cellulaire à partir de cette cellule.

30. Procédé conforme à la revendication 29, qui comporte en outre les étapes suivantes :
viii) cultiver une lignée cellulaire obtenue selon un procédé conforme à la revendication 29, dans des conditions qui permettent l'expression de ladite protéine intéressante ;
ix) et recueillir ladite protéine intéressante.

31. Procédé conforme à la revendication 30, qui comporte en outre une étape de purification de ladite protéine intéressante.

32. Procédé conforme à la revendication 31, qui comporte en outre une étape de formulation de ladite protéine intéressante en une composition pharmaceutique.


### SEQUENCE LISTING

<110> Applied Research Systems ARS Holding N.V.
<120> THE LUPAC BIFUNCTIONAL MARKER AND ITS USE IN PROTEIN PRODUCTION
<130> 955 EP
<160> 9
<170> PatentIn version 3.1
<210> 1
   <211> 2247
   <212> DNA
   <213> Artificial
<220>
   <223> Lupac
<220>
   <221> misc_feature
   <222> (1)..(1647)
   <223> Sequence from photinus pyralis luciferase
<220>
   <221> CDS
   <222> (1)..(2247)
   <223>
<220>
   <221> misc_feature
   <222> (1647)..(1653)
   <223> Fusion sequence
<220>
   <221> misc_feature
   <222> (1654)..(2247)
   <223> Sequence from Streptomyces alboniger pac
<400> 1
   atg gaa gac gcc aaa aac ata aag aaa ggc ccg gcg cca ttc tat ccg    48
   Met Glu Asp Ala Lys Asn Ile Lys Lys Gly Pro Ala Pro Phe Tyr Pro
   1               5                   10                  15
   ctg gaa gat gga acc gct gga gag caa ctg cat aag gct atg aag aga    96
   Leu Glu Asp Gly Thr Ala Gly Glu Gln Leu His Lys Ala Met Lys Arg
               20                  25                  30
   tac gcc ctg gtt cct gga aca att gct ttt aca gat gca cat atc gag    144
   Tyr Ala Leu Val Pro Gly Thr Ile Ala Phe Thr Asp Ala His Ile Glu
           35                  40                  45
   gtg gac atc act tac gct gag tac ttc gaa atg tcc gtt cgg ttg gca    192
   Val Asp Ile Thr Tyr Ala Glu Tyr Phe Glu Met Ser Val Arg Leu Ala
       50                  55                  60
   gaa gct atg aaa cga tat ggg ctg aat aca aat cac aga atc gtc gta    240
   Glu Ala Met Lys Arg Tyr Gly Leu Asn Thr Asn His Arg Ile Val Val
   65                  70                  75                  80
   tgc agt gaa aac tct ctt caa ttc ttt atg ccg gtg ttg ggc gcg tta    288
   Cys Ser Glu Asn Ser Leu Gln Phe Phe Met Pro Val Leu Gly Ala Leu
                   85                  90                  95
   ttt atc gga gtt gca gtt gcg ccc gcg aac gac att tat aat gaa cgt    336
   Phe Ile Gly Val Ala Val Ala Pro Ala Asn Asp Ile Tyr Asn Glu Arg
               100                 105                 110
   gaa ttg ctc aac agt atg ggc att tcg cag cct acc gtg gtg ttc gtt      384
   Glu Leu Leu Asn Ser Met Gly Ile Ser Gln Pro Thr Val Val Phe Val
           115                 120                 125
   tcc aaa aag ggg ttg caa aaa att ttg aac gtg caa aaa aag ctc cca      432
   Ser Lys Lys Gly Leu Gln Lys Ile Leu Asn Val Gln Lys Lys Leu Pro
       130                 135                 140
   atc atc caa aaa att att atc atg gat tct aaa acg gat tac cag gga      480
   Ile Ile Gln Lys Ile Ile Ile Met Asp Ser Lys Thr Asp Tyr Gln Gly
   145                 150                 155                 160
   ttt cag tcg atg tac acg ttc gtc aca tct cat cta cct ccc ggt ttt      528
   Phe Gln Ser Met Tyr Thr Phe Val Thr Ser His Leu Pro Pro Gly Phe
                   165                 170                 175
   aat gaa tac gat ttt gtg cca gag tcc ttc gat agg gac aag aca att      576
   Asn Glu Tyr Asp Phe Val Pro Glu Ser Phe Asp Arg Asp Lys Thr Ile
               180                 185                 190
   gca ctg atc atg aac tcc tct gga tct act ggt ctg cct aaa ggt gtc      624
   Ala Leu Ile Met Asn Ser Ser Gly Ser Thr Gly Leu Pro Lys Gly Val
           195                 200                 205
   gct ctg cct cat aga act gcc tgc gtg aga ttc tcg cat gcc aga gat      672
   Ala Leu Pro His Arg Thr Ala Cys Val Arg Phe Ser His Ala Arg Asp
       210                 215                 220
   cct att ttt ggc aat caa atc att ccg gat act gcg att tta agt gtt      720
   Pro Ile Phe Gly Asn Gln Ile Ile Pro Asp Thr Ala Ile Leu Ser Val
   225                 230                 235                 240
   gtt cca ttc cat cac ggt ttt gga atg ttt act aca ctc gga tat ttg      768
   Val Pro Phe His His Gly Phe Gly Met Phe Thr Thr Leu Gly Tyr Leu
                   245                 250                 255
   ata tgt gga ttt cga gtc gtc tta atg tat aga ttt gaa gaa gag ctg      816
   Ile Cys Gly Phe Arg Val Val Leu Met Tyr Arg Phe Glu Glu Glu Leu
               260                 265                 270
   ttt ctg agg agc ctt cag gat tac aag att caa agt gcg ctg ctg gtg      864
   Phe Leu Arg Ser Leu Gln Asp Tyr Lys Ile Gln Ser Ala Leu Leu Val
           275                 280                 285
   cca acc cta ttc tcc ttc ttc gcc aaa agc act ctg att gac aaa tac      912
   Pro Thr Leu Phe Ser Phe Phe Ala Lys Ser Thr Leu Ile Asp Lys Tyr
       290                 295                 300
   gat tta tct aat tta cac gaa att gct tct ggt ggc gct ccc ctc tct      960
   Asp Leu Ser Asn Leu His Glu Ile Ala Ser Gly Gly Ala Pro Leu Ser
   305                 310                 315                 320
   aag gaa gtc ggg gaa gcg gtt gcc aag agg ttc cat ctg cca ggt atc     1008
   Lys Glu Val Gly Glu Ala Val Ala Lys Arg Phe His Leu Pro Gly Ile
                   325                 330                 335
   agg caa gga tat ggg ctc act gag act aca tca gct att ctg att aca     1056
   Arg Gln Gly Tyr Gly Leu Thr Glu Thr Thr Ser Ala Ile Leu Ile Thr
               340                 345                 350
   ccc gag ggg gat gat aaa ccg ggc gcg gtc ggt aaa gtt gtt cca ttt     1104
   Pro Glu Gly Asp Asp Lys Pro Gly Ala Val Gly Lys Val Val Pro Phe
           355                 360                 365
   ttt gaa gcg aag gtt gtg gat ctg gat acc ggg aaa acg ctg ggc gtt     1152
   Phe Glu Ala Lys Val Val Asp Leu Asp Thr Gly Lys Thr Leu Gly Val
   370 375 380
   aat caa aga ggc gaa ctg tgt gtg aga ggt cct atg att atg tcc ggt 1200
   Asn Gln Arg Gly Glu Leu Cys Val Arg Gly Pro Met Ile Met Ser Gly
   385 390 395 400
   tat gta aac aat ccg gaa gcg acc aac gcc ttg att gac aag gat gga 1248
   Tyr Val Asn Asn Pro Glu Ala Thr Asn Ala Leu Ile Asp Lys Asp Gly
   405 410 415
   tgg cta cat tct gga gac ata gct tac tgg gac gaa gac gaa cac ttc 1296
   Trp Leu His Ser Gly Asp Ile Ala Tyr Trp Asp Glu Asp Glu His Phe
   420 425 430
   ttc atc gtt gac cgc ctg aag tct ctg att aag tac aaa ggc tat cag 1344
   Phe Ile Val Asp Arg Leu Lys Ser Leu Ile Lys Tyr Lys Gly Tyr Gln
   435 440 445
   gtg gct ccc gct gaa ttg gaa tcc atc ttg ctc caa cac ccc aac atc 1392
   Val Ala Pro Ala Glu Leu Glu Ser Ile Leu Leu Gln His Pro Asn Ile
   450 455 460
   ttc gac gca ggt gtc gca ggt ctt ccc gac gat gac gcc ggt gaa ctt 1440
   Phe Asp Ala Gly Val Ala Gly Leu Pro Asp Asp Asp Ala Gly Glu Leu
   465 470 475 480
   ccc gcc gcc gtt gtt gtt ttg gag cac gga aag acg atg acg gaa aaa 1488
   Pro Ala Ala Val Val Val Leu Glu His Gly Lys Thr Met Thr Glu Lys
   485 490 495
   gag atc gtg gat tac gtc gcc agt caa gta aca acc gcg aaa aag ttg 1536
   Glu Ile Val Asp Tyr Val Ala Ser Gln Val Thr Thr Ala Lys Lys Leu
   500 505 510
   cgc gga gga gtt gtg ttt gtg gac gaa gta ccg aaa ggt ctt acc gga 1584
   Arg Gly Gly Val Val Phe Val Asp Glu Val Pro Lys Gly Leu Thr Gly
   515 520 525
   aaa ctc gac gca aga aaa atc aga gag atc ctc ata aag gcc aag aag 1632
   Lys Leu Asp Ala Arg Lys Ile Arg Glu Ile Leu Ile Lys Ala Lys Lys
   530 535 540
   ggc gga aag atc gcc gtg acc gag tac aag ccc acg gtg cgc ctc gcc 1680
   Gly Gly Lys Ile Ala Val Thr Glu Tyr Lys Pro Thr Val Arg Leu Ala
   545 550 555 560
   acc cgc gac gac gtc ccc cgg gcc gta cgc acc ctc gcc gcc gcg ttc 1728
   Thr Arg Asp Asp Val Pro Arg Ala Val Arg Thr Leu Ala Ala Ala Phe
   565 570 575
   gcc gac tac ccc gcc acg cgc cac acc gtc gac ccg gac cgc cac atc 1776
   Ala Asp Tyr Pro Ala Thr Arg His Thr Val Asp Pro Asp Arg His Ile
   580 585 590
   gag cgg gtc acc gag ctg caa gaa ctc ttc ctc acg cgc gtc ggg ctc 1824
   Glu Arg Val Thr Glu Leu Gln Glu Leu Phe Leu Thr Arg Val Gly Leu
   595 600 605
   gac atc ggc aag gtg tgg gtc gcg gac gac ggc gcc gcg gtg gcg gtc 1872
   Asp Ile Gly Lys Val Trp Val Ala Asp Asp Gly Ala Ala Val Ala Val
   610 615 620
   tgg acc acg ccg gag agc gtc gaa gcg ggg gcg gtg ttc gcc gag atc 1920
   Trp Thr Thr Pro Glu Ser Val Glu Ala Gly Ala Val Phe Ala Glu Ile
   625 630 635 640
   ggc ccg cgc atg gcc gag ttg agc ggt tcc cgg ctg gcc gcg cag caa 1968
   Gly Pro Arg Met Ala Glu Leu Ser Gly Ser Arg Leu Ala Ala Gln Gln
   645 650 655
   cag atg gaa ggc ctc ctg gcg ccg cac cgg ccc aag gag ccc gcg tgg      2016
   Gln Met Glu Gly Leu Leu Ala Pro His Arg Pro Lys Glu Pro Ala Trp
               660                 665                 670
   ttc ctg gcc acc gtc ggc gtc tcg ccc gac cac cag ggc aag ggt ctg      2064
   Phe Leu Ala Thr Val Gly Val Ser Pro Asp His Gln Gly Lys Gly Leu
           675                 680                 685
   ggc agc gcc gtc gtg ctc ccc gga gtg gag gcg gcc gag cgc gcc ggg      2112
   Gly Ser Ala Val Val Leu Pro Gly Val Glu Ala Ala Glu Arg Ala Gly
       690                 695                 700
   gtg ccc gcc ttc ctg gag acc tcc gcg ccc cgc aac ctc ccc ttc tac      2160
   Val Pro Ala Phe Leu Glu Thr Ser Ala Pro Arg Asn Leu Pro Phe Tyr
   705                 710                 715                 720
   gag cgg ctc ggc ttc acc gtc acc gcc gac gtc gag gtg ccc gaa gga      2208
   Glu Arg Leu Gly Phe Thr Val Thr Ala Asp Val Glu Val Pro Glu Gly
                   725                 730                 735
   ccg cgc acc tgg tgc atg acc cgc aag ccc ggt gcc tga                  2247
   Pro Arg Thr Trp Cys Met Thr Arg Lys Pro Gly Ala
               740                 745
<210> 2
   <211> 748
   <212> PRT
   <213> Artificial
<220>
   <223> Lupac
<400> 2
   Met Glu Asp Ala Lys Asn Ile Lys Lys Gly Pro Ala Pro Phe Tyr Pro
   1               5                   10                  15
   Leu Glu Asp Gly Thr Ala Gly Glu Gln Leu His Lys Ala Met Lys Arg
               20                  25                  30
   Tyr Ala Leu Val Pro Gly Thr Ile Ala Phe Thr Asp Ala His Ile Glu
           35                  40                  45
   Val Asp Ile Thr Tyr Ala Glu Tyr Phe Glu Met Ser Val Arg Leu Ala
       50                  55                  60
   Glu Ala Met Lys Arg Tyr Gly Leu Asn Thr Asn His Arg Ile Val Val
   65                  70                  75                  80
   Cys Ser Glu Asn Ser Leu Gln Phe Phe Met Pro Val Leu Gly Ala Leu
                   85                  90                  95
   Phe Ile Gly Val Ala Val Ala Pro Ala Asn Asp Ile Tyr Asn Glu Arg
               100                 105                 110
   Glu Leu Leu Asn Ser Met Gly Ile Ser Gln Pro Thr Val Val Phe Val
           115                 120                 125
   Ser Lys Lys Gly Leu Gln Lys Ile Leu Asn Val Gln Lys Lys Leu Pro
   130 135 140
   Ile Ile Gln Lys Ile Ile Ile Met Asp Ser Lys Thr Asp Tyr Gln Gly
   145 150 155 160
   Phe Gln Ser Met Tyr Thr Phe Val Thr Ser His Leu Pro Pro Gly Phe
   165 170 175
   Asn Glu Tyr Asp Phe Val Pro Glu Ser Phe Asp Arg Asp Lys Thr Ile
   180 185 190
   Ala Leu Ile Met Asn Ser Ser Gly Ser Thr Gly Leu Pro Lys Gly Val
   195 200 205
   Ala Leu Pro His Arg Thr Ala Cys Val Arg Phe Ser His Ala Arg Asp
   210 215 220
   Pro Ile Phe Gly Asn Gln Ile Ile Pro Asp Thr Ala Ile Leu Ser Val
   225 230 235 240
   Val Pro Phe His His Gly Phe Gly Met Phe Thr Thr Leu Gly Tyr Leu
   245 250 255
   Ile Cys Gly Phe Arg Val Val Leu Met Tyr Arg Phe Glu Glu Glu Leu
   260 265 270
   Phe Leu Arg Ser Leu Gln Asp Tyr Lys Ile Gln Ser Ala Leu Leu Val
   275 280 285
   Pro Thr Leu Phe Ser Phe Phe Ala Lys Ser Thr Leu Ile Asp Lys Tyr
   290 295 300
   Asp Leu Ser Asn Leu His Glu Ile Ala Ser Gly Gly Ala Pro Leu Ser
   305 310 315 320
   Lys Glu Val Gly Glu Ala Val Ala Lys Arg Phe His Leu Pro Gly Ile
   325 330 335
   Arg Gln Gly Tyr Gly Leu Thr Glu Thr Thr Ser Ala Ile Leu Ile Thr
   340 345 350
   Pro Glu Gly Asp Asp Lys Pro Gly Ala Val Gly Lys Val Val Pro Phe
   355 360 365
   Phe Glu Ala Lys Val Val Asp Leu Asp Thr Gly Lys Thr Leu Gly Val
   370 375 380
   Asn Gln Arg Gly Glu Leu Cys Val Arg Gly Pro Met Ile Met Ser Gly
   385 390 395 400
   Tyr Val Asn Asn Pro Glu Ala Thr Asn Ala Leu Ile Asp Lys Asp Gly
   405 410 415
   Trp Leu His Ser Gly Asp Ile Ala Tyr Trp Asp Glu Asp Glu His Phe
   420 425 430
   Phe Ile Val Asp Arg Leu Lys Ser Leu Ile Lys Tyr Lys Gly Tyr Gln
   435 440 445
   Val Ala Pro Ala Glu Leu Glu Ser Ile Leu Leu Gln His Pro Asn Ile
   450 455 460
   Phe Asp Ala Gly Val Ala Gly Leu Pro Asp Asp Asp Ala Gly Glu Leu
   465 470 475 480
   Pro Ala Ala Val Val Val Leu Glu His Gly Lys Thr Met Thr Glu Lys
   485 490 495
   Glu Ile Val Asp Tyr Val Ala Ser Gln Val Thr Thr Ala Lys Lys Leu
   500 505 510
   Arg Gly Gly Val Val Phe Val Asp Glu Val Pro Lys Gly Leu Thr Gly
   515 520 525
   Lys Leu Asp Ala Arg Lys Ile Arg Glu Ile Leu Ile Lys Ala Lys Lys
   530 535 540
   Gly Gly Lys Ile Ala Val Thr Glu Tyr Lys Pro Thr Val Arg Leu Ala
   545 550 555 560
   Thr Arg Asp Asp Val Pro Arg Ala Val Arg Thr Leu Ala Ala Ala Phe
   565 570 575
   Ala Asp Tyr Pro Ala Thr Arg His Thr Val Asp Pro Asp Arg His Ile
   580 585 590
   Glu Arg Val Thr Glu Leu Gln Glu Leu Phe Leu Thr Arg Val Gly Leu
   595 600 605
   Asp Ile Gly Lys Val Trp Val Ala Asp Asp Gly Ala Ala Val Ala Val
   610 615 620
   Trp Thr Thr Pro Glu Ser Val Glu Ala Gly Ala Val Phe Ala Glu Ile
   625 630 635 640
   Gly Pro Arg Met Ala Glu Leu Ser Gly Ser Arg Leu Ala Ala Gln Gln
   645 650 655
   Gln Met Glu Gly Leu Leu Ala Pro His Arg Pro Lys Glu Pro Ala Trp
   660 665 670
   Phe Leu Ala Thr Val Gly Val Ser Pro Asp His Gln Gly Lys Gly Leu
   675 680 685
   Gly Ser Ala Val Val Leu Pro Gly Val Glu Ala Ala Glu Arg Ala Gly
   690 695 700
   Val Pro Ala Phe Leu Glu Thr Ser Ala Pro Arg Asn Leu Pro Phe Tyr
   705 710 715 720
   Glu Arg Leu Gly Phe Thr Val Thr Ala Asp Val Glu Val Pro Glu Gly
   725 730 735
   Pro Arg Thr Trp Cys Met Thr Arg Lys Pro Gly Ala
   740 745
<210> 3
   <211> 25
   <212> DNA
   <213> Artificial
<220>
   <223> Primer
<400> 3
   agtaggtcct atgattatgt ccggt 25
<210> 4
   <211> 22
   <212> DNA
   <213> Artificial
<220>
   <223> Primer
<400> 4
   cacggcgatc tttccgccct tc 22
<210> 5
   <211> 42
   <212> DNA
   <213> Artificial
<220>
   <223> Primer
<400> 5
   gaagggcgga aagatcgccg tgaccgagta caagcccacg gt 42
<210> 6
   <211> 23
   <212> DNA
   <213> Artificial
<220>
   <223> Primer
<400> 6
   tcaggcaccg ggcttgcggg tca 23
<210> 7
   <211> 1394
   <212> DNA
   <213> murine cytomegalovirus
<400> 7
<210> 8
   <211> 550
   <212> PRT
   <213> Photinus pyralis
<400> 8
   Met Glu Asp Ala Lys Asn Ile Lys Lys Gly Pro Ala Pro Phe Tyr Pro
   1               5                   10                  15
   Leu Glu Asp Gly Thr Ala Gly Glu Gln Leu His Lys Ala Met Lys Arg
               20                  25                  30
   Tyr Ala Leu Val Pro Gly Thr Ile Ala Phe Thr Asp Ala His Ile Glu
           35                  40                  45
   Val Asn Ile Thr Tyr Ala Glu Tyr Phe Glu Met Ser Val Arg Leu Ala
       50                  55                  60
   Glu Ala Met Lys Arg Tyr Gly Leu Asn Thr Asn His Arg Ile Val Val
   65                  70                  75                  80
   Cys Ser Glu Asn Ser Leu Gln Phe Phe Met Pro Val Leu Gly Ala Leu
                   85                  90                  95
   Phe Ile Gly Val Ala Val Ala Pro Ala Asn Asp Ile Tyr Asn Glu Arg
               100                 105                 110
   Glu Leu Leu Asn Ser Met Asn Ile Ser Gln Pro Thr Val Val Phe Val
           115                 120                 125
   Ser Lys Lys Gly Leu Gln Lys Ile Leu Asn Val Gln Lys Lys Leu Pro
       130                 135                 140
   Ile Ile Gln Lys Ile Ile Ile Met Asp Ser Lys Thr Asp Tyr Gln Gly
   145                 150                 155                 160
   Phe Gln Ser Met Tyr Thr Phe Val Thr Ser His Leu Pro Pro Gly Phe
                   165                 170                 175
   Asn Glu Tyr Asp Phe Val Pro Glu Ser Phe Asp Arg Asp Lys Thr Ile
               180                 185                 190
   Ala Leu Ile Met Asn Ser Ser Gly Ser Thr Gly Leu Pro Lys Gly Val
           195                 200                 205
   Ala Leu Pro His Arg Thr Ala Cys Val Arg Phe Ser His Ala Arg Asp
       210                 215                 220
   Pro Ile Phe Gly Asn Gln Ile Ile Pro Asp Thr Ala Ile Leu Ser Val
   225                 230                 235                 240
   Val Pro Phe His His Gly Phe Gly Met Phe Thr Thr Leu Gly Tyr Leu
                   245                 250                 255
   Ile Cys Gly Phe Arg Val Val Leu Met Tyr Arg Phe Glu Glu Glu Leu
               260                 265                 270
   Phe Leu Arg Ser Leu Gln Asp Tyr Lys Ile Gln Ser Ala Leu Leu Val
           275                 280                 285
   Pro Thr Leu Phe Ser Phe Phe Ala Lys Ser Thr Leu Ile Asp Lys Tyr
       290                 295                 300
   Asp Leu Ser Asn Leu His Glu Ile Ala Ser Gly Gly Ala Pro Leu Ser
   305                 310                 315                 320
   Lys Glu Val Gly Glu Ala Val Ala Lys Arg Phe His Leu Pro Gly Ile
   325 330 335
   Arg Gln Gly Tyr Gly Leu Thr Glu Thr Thr Ser Ala Ile Leu Ile Thr
   340 345 350
   Pro Glu Gly Asp Asp Lys Pro Gly Ala Val Gly Lys Val Val Pro Phe
   355 360 365
   Phe Glu Ala Lys Val Val Asp Leu Asp Thr Gly Lys Thr Leu Gly Val
   370 375 380
   Asn Gln Arg Gly Glu Leu Cys Val Arg Gly Pro Met Ile Met Ser Gly
   385 390 395 400
   Tyr Val Asn Asn Pro Glu Ala Thr Asn Ala Leu Ile Asp Lys Asp Gly
   405 410 415
   Trp Leu His Ser Gly Asp Ile Ala Tyr Trp Asp Glu Asp Glu His Phe
   420 425 430
   Phe Ile Val Asp Arg Leu Lys Ser Leu Ile Lys Tyr Lys Gly Tyr Gln
   435 440 445
   Val Ala Pro Ala Glu Leu Glu Ser Ile Leu Leu Gln His Pro Asn Ile
   450 455 460
   Phe Asp Ala Gly Val Ala Gly Leu Pro Asp Asp Asp Ala Gly Glu Leu
   465 470 475 480
   Pro Ala Ala Val Val Val Leu Glu His Gly Lys Thr Met Thr Glu Lys
   485 490 495
   Glu Ile Val Asp Tyr Val Ala Ser Gln Val Thr Thr Ala Lys Lys Leu
   500 505 510
   Arg Gly Gly Val Val Phe Val Asp Glu Val Pro Lys Gly Leu Thr Gly
   515 520 525
   Lys Leu Asp Ala Arg Lys Ile Arg Glu Ile Leu Ile Lys Ala Lys Lys
   530 535 540
   Gly Gly Lys Ser Lys Leu
   545 550
<210> 9
   <211> 199
   <212> PRT
   <213> Streptomyces alboniger
<400> 9
   Met Thr Glu Tyr Lys Pro Thr Val Arg Leu Ala Thr Arg Asp Asp Val
   1               5                   10                  15
   Pro Arg Ala Val Arg Thr Leu Ala Ala Ala Phe Ala Asp Tyr Pro Ala
               20                  25                  30
   Thr Arg His Thr Val Asp Pro Asp Arg His Ile Glu Arg Val Thr Glu
           35                  40                  45
   Leu Gln Glu Leu Phe Leu Thr Arg Val Gly Leu Asp Ile Gly Lys Val
       50                  55                  60
   Trp Val Ala Asp Asp Gly Ala Ala Val Ala Val Trp Thr Thr Pro Glu
   65                  70                  75                  80
   Ser Val Glu Ala Gly Ala Val Phe Ala Glu Ile Gly Pro Arg Met Ala
                   85                  90                  95
   Glu Leu Ser Gly Ser Arg Leu Ala Ala Gln Gln Gln Met Glu Gly Leu
               100                 105                 110
   Leu Ala Pro His Arg Pro Lys Glu Pro Ala Trp Phe Leu Ala Thr Val
           115                 120                 125
   Gly Val Ser Pro Asp His Gln Gly Lys Gly Leu Gly Ser Ala Val Val
       130                 135                 140
   Leu Pro Gly Val Glu Ala Ala Glu Arg Ala Gly Val Pro Ala Phe Leu
   145                 150                 155                 160
   Glu Thr Ser Ala Pro Arg Asn Leu Pro Phe Tyr Glu Arg Leu Gly Phe
                   165                 170                 175
   Thr Val Thr Ala Asp Val Glu Val Pro Glu Gly Pro Arg Thr Trp Cys
               180                 185                 190
   Met Thr Arg Lys Pro Gly Ala
           195